# EUROPEAN PATENT SPECIFICATION

(11) **EP 0 541 693 B1**
(45) Date of publication and mention of the grant of the patent: **09.12.1998**
(21) Application number: 91915000.3
(22) Date of filing: 29.07.1991
(51) Int. Cl.: C07H 1/00, C07H 21/00, C12Q 1/68

(54) **LARGE COMB-TYPE BRANCHED POLYNUCLEOTIDES**
GROSSE KAMMFÖRMIG VERZWEIGTE POLYNUKLEOTIDE
POLYNUCLEOTIDES RAMIFIES DE TYPE GRAND PEIGNE

(30) Priority: 27.07.1990 US 558897
(43) Date of publication of application: 19.05.1993
(73) Proprietor: CHIRON CORPORATION, Emeryville, California 94608 (US)
(72) Inventor: URDEA, Michael, S., Alamo, CA 94507 (US); HORN, Thomas, Berkeley, CA 94708 (US); CHANG, Chu-An, Piedmont, CA 94611 (US)
(74) Representative: Goldin, Douglas Michael (GB)
(86) International application number: US9105363
(87) International publication number: WO9202526

(56) References cited:
- EP-A- 0 138 357
- EP-A- 0 225 807
- EP-A- 0 317 077
- US-A- 4 755 458
- US-A- 4 843 122
- US-A- 4 910 300
- Chemica Scripta, Volume 20, issued 1982, NEIL BALGOBIN et al., "A Novel Strategy for the chemical synthesis of DNA and RNA fragments using 2-oxymethylene anthraquinone (MAQ) as a 3-Terminal Phosphate Protecting Group", pages, see entire document.

## Description

### Technical Field

This invention is in the field of nucleic acid chemistry and biochemical assays. More particularly, it concerns large, comb-type branched polynucleotides that are useful as amplification multimers in nucleic acid hybridization assays.

### Background

Nucleic acid hybridization assays are commonly used in genetic research, biomedical research and clinical diagnostics. In a basic nucleic acid hybridization assay, single-stranded analyte nucleic acid is hybridized to a labeled single-stranded nucleic acid probe and resulting labeled duplexes are detected. Variations of this basic scheme have been developed to facilitate separation of the duplexes to be detected from extraneous materials and/or to amplify the signal that is detected. One method for amplifying the signal that is detected is described in commonly owned European Patent Application No. 88309697.6 as published under No. EP-A 0317077. It amplifies the signal through the use of amplification multimers. These multimers are polynucleotides that are constructed to have a first segment that hybridizes specifically to the analyte nucleic acid or a strand of nucleic acid bound to the analyte and iterations of a second segment that hybridizes specifically to a labeled probe. The amplification is theoretically proportional to the number of iterations of the second segment. The multimers may be either linear or branched. Two general types of branched multimers are described: fork and comb.

In testing the two types of branched multimers it was found that forked structures of greater than about eight branches exhibited steric hindrance which inhibited binding of labeled probes to the multimer. On the other hand the comb structures exhibited no steric problems and were thus determined to be the preferred type of branched multimer. Unfortunately, however, repeated attempts to make comb structures having more than about 10 branch sites were unsuccessful. Applicants have now developed procedures for producing large comb-type branched multimers. These large comb structures permit a greater degree of amplification than possible previously.

### Disclosure of the Invention

One aspect of the invention is a large comb-type branched polynucleotide comprising:
(a) a polynucleotide backbone having:
   (i) at least 15 multifunctional nucleotides, each of which defines a sidechain site and
   (ii) a first single-stranded oligonucleotide unit that is capable of binding specifically to a first single-stranded polynucleotide sequence of interest; and
(b) pendant polynucleotide sidechains extending from said multifunctional nucleotides, at least 70% of which comprise iterations of a second single-stranded oligonucleotide unit that is capable of binding specifically to a second single-stranded polynucleotide sequence of interest, the total number of iterations in all sidechains being at least 20.

Another aspect of the invention is a process for making a large comb-type branched polynucleotide useful as an amplification multimer in a nucleic acid hybridization assay comprising:
(a) synthesizing a single-stranded polynucleotide backbone comprising:
   (i) at least about 15 multifunctional nucleotides, each of which has a protected functional group that serves as a site for sidechain nucleotide extension and
   (ii) a first ligation site segment;
(b) deprotecting said functional groups;
(c) extending each of said sites at least about five nucleotides to provide second ligation site segments;
(d) ligating a first single-stranded oligonucleotide unit to the first ligation site, said first single-stranded oligonucleotide unit being capable of binding specifically to a first single-stranded nucleic acid sequence of interest; and
(e) ligating second single-stranded oligonucleotide units to the second ligation site segments, said second single-stranded oligonucleotide units comprising iterations of a sequence that is capable of binding specifically to a second single-stranded oligonucleotide of interest.

Still another aspect of the invention is an alternative process for making a large comb-type branch polynucleotide useful as an amplification multimer in a nucleic acid hybridization assay comprising:
(a) synthesizing a single-stranded polynucleotide backbone comprising:
   (i) at least about 15 multifunctional nucleotides, each of which has a protected functional group that serves as a site for sidechain nucleotide extension and
   (ii) a first single-stranded oligonucleotide unit that is capable of binding specifically to a first single-stranded polynucleotide sequence of interest;
(b) deprotecting said functional groups;
(c) extending each of said sites at least about five nucleotides to provide ligation site segments; and
(d) ligating second single-stranded oligonucleotide units to the ligation site segments said second single-stranded oligonucleotide units comprising iterations of a sequence that is capable of binding to a second single-stranded oligonucleotide of interest.

Yet another aspect of the invention is the use of these large comb-type branched polynucleotides in nucleic acid hybridization assays. In these assays:
(a) the branched polynucleotide is hybridized via the first oligonucleotide unit to single-stranded analyte nucleic acid bound to a solid phase or to a single-stranded oligonucleotide bound to the analyte;
(b) unbound branched polynucleotide is removed;
(c) single-stranded labeled oligonucleotide is hybridized to the branched polynucleotide via the second oligonucleotide units;
(d) unbound labeled oligonucleotide is removed; and
(e) the presence of label bound to the branched polynucleotide is detected.

### Modes for Carrying Out the Invention

### Definitions

"Large" as used herein to describe the comb-type branched polynucleotides of the invention intends a molecule having at least about 15 branch sites and at least about 20 iterations of the labeled probe binding sequence.

"Comb-type" as used herein to describe the structure of the branched polynucleotides of the invention intends a polynucleotide having a linear backbone with a multiplicity of sidechains extending from the backbone.

A "multifunctional" or "modified" nucleotide intends a nucleotide monomer which may be stably incorporated into a polynucleotide having an additional functional group, (preferably a cytosine in which the 4-position is modified to provide a functional hydroxy group), to which a nucleotide may be covalently bonded to form a sidechain.

A "cleavable linker molecule" intends a molecule that may be stably incorporated into a polynucleotide chain and which includes a covalent bond that may be broken or cleaved by chemical treatment or physical treatment such as by irradiation.

An "amplification multimer" intends a polynucleotide that is capable of hybridizing directly or indirectly to analyte nucleic acid and to a multiplicity of labeled probes.

### Characterization of Large Comb-Type Branched Polynucleotides

The polynucleotide multimers of the invention are composed of a linear backbone and pendant sidechains. The backbone includes a segment that provides a specific hybridization site for analyte nucleic acid or nucleic acid bound to the analyte; whereas the pendant sidechains include iterations of a segment that provide specific hybridization sites for a labeled probe.

Preferred embodiments of these comb-type polynucleotide multimers may be represented by the following schematic formula: 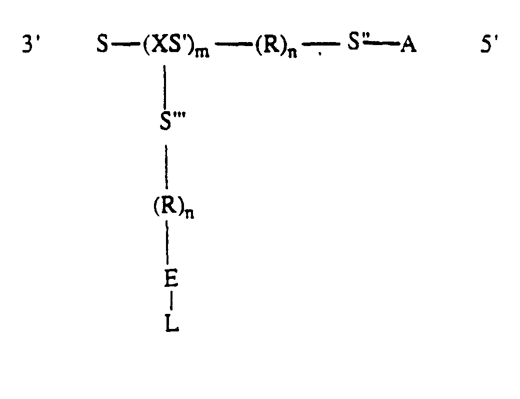 where S is a first spacer segment of at least about 15 nucleotides, preferably about 15 to 50 nucleotides, X is a multifunctional nucleotide that provides a branch site, S' is a branching site spacer segment of 0 to about 15 nucleotides, preferably 0 to 10 nucleotides, m is an integer equal to or greater than 15, preferably in the range of 15 to 100, R is a cleavable linker molecule, n is 0 or 1, S" is a second spacer segment of about 0 to 10 nucleotides, preferably 5 to 10 nucleotides, A is a segment that is capable of hybridizing specifically to analyte nucleic acid or nucleic acid bound to analyte, S''' is a third spacer segment of 0 to 10 nucleotides, E is an oligonucleotide extension of 5 to 10 nucleotides and L is a segment containing 2 to 10 iterations, preferably 3 to 6 iterations, of a nucleotide sequence that is capable of hybridizing specifically to a labeled oligonucleotide probe.

The entire backbone of the multimer or the portion thereof from S to S", inclusive, and the portion of the sidechain excluding L will typically be synthesized chemically as an integral unit using conventional automated solid-phase oligonucleotide synthesis chemistry and equipment. In this regard, the spacer segment S serves to space the portion of the molecule that contains the branching sites from the solid phase (the 3' end of S is bound to the surface of the solid phase).

The modified nucleotides or branching monomers designated X in the above formula are multifunctional nucleotides in which one functional group is used for sidechain extension and the others are used for backbone bonds. Examples of multifunctional nucleotides are described in EPA 0317077.

These modified nucleotides are preferably of the formula: 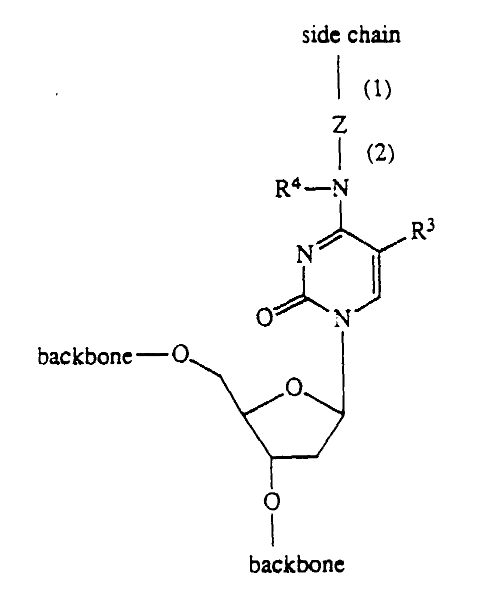 where R³ is hydrogen, methyl, I, Br or F, R⁴ is hydrogen or methyl, Z is selected from the group consisting of

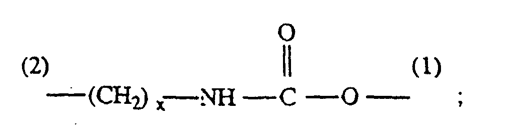

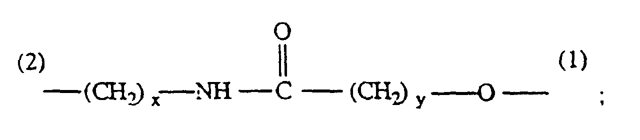

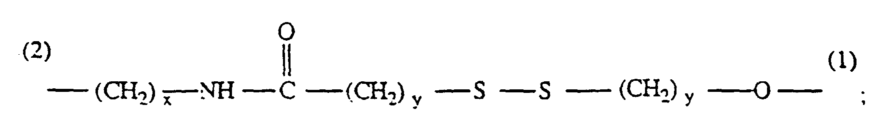

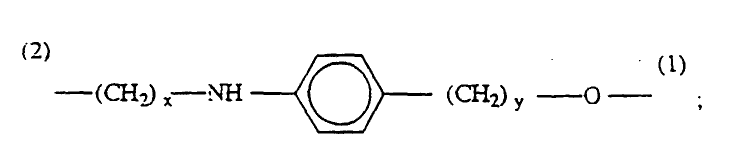

⁽²⁾―(CH₂―CH₂―O)ₓ―⁽¹⁾ ;

and

⁽²⁾―(CH₂)ₓ―O―⁽¹⁾,

wherein x and y may be the same or different and are integers in the range of 1 to 8, inclusive. (The designations "(1)" and "(2)" at the Z linkage indicate the orientation of the Z linker moiety; (1) indicates the direction away from the backbone, (2) indicates the direction towards the backbone).

As indicated, the spacer seqment S' is optional and may be used, if desired, to space each branch site from preceding/succeeding flanking branch sites or a series of adjacent branch sites from flanking series of branch sites. The second spacer segment S" is also optional and may be employed to space the branched portion of the molecule from the segment A to which the analyte is ultimately bound. Such spacing has been found to improve the binding between the analyte and the multimer. Likewise, the third spacer segment S is optional. It is preferably polyT.

Segment A has a sequence and length that permits it to bind specifically and stably to the analyte or nucleic acid bound to the analyte. In order to achieve such specificity and stability segment A will normally be 15 to 50, preferably 15 to 30, nucleotides in length and have a GC content in the range of 40% to 60%. The specific length and sequence of this segment will, of course, vary depending upon the nucleic acid to which it is intended to hybridize.

Segment E is a sidechain extension that is chemically synthesized using automated solid-phase oligonucleotide synthesis equipment and techniques. It is typically about 5 to 10 nucleotides in length and serves as a site to which segment L may be ligated enzymatically.

Segment L comprises iterations of an oligomer unit that is capable of hybridizing specifically and stably to a labeled oligonucleotide probe. These units are also typically 15 to 150, preferably 20 to 120, nucleotides in length and have a GC content in the range of 40% and 60%. Each L segment will normally contain 2 to 10 iterations of the unit, preferably 3 to 6 iterations. Some sidechains may not include an L segment. Normally at least about 50% of the sidechains, preferaby at least about 70% of the sidechains, will include an L segment.

The cleavable linker molecules (R) in the backbone and/or sidechains are optional, but preferred. They provide selectable cleavage sites so that samples of the large, comb-type polynucleotide may be cleaved for analysis and characterization purposes. In this regard it is preferred that there be cleavage sites in each sidechain and a cleavage site just 5' of the 5'-most branch site. Examples of cleavable linker molecules that may be incorporated into the polynucleotides are disclosed in EPA 0317077 and in the examples, infra.

### Synthesis of Large comb-Type Branched Multimers

The polynucleotides of the invention are assembled using a combination of solid phase direct oligonucleotide synthesis and enzymatic ligation methods.

The comb body, which includes the 3' spacer (S), branching sites (X), optionally the S' S" and S''' segments, the A segment, optionally the desirable linker molecules (R) and the sidechain extension E, is synthesized by automated solid phase oligonucleotide synthesis techniques. A preferred solid phase is controlled pore glass of at least 2000 Å pore size. In this synthesis spacer segment S is extended from the solid phase. For convenience, this segment is polyT. The multifunctional nucleotides that provide the branch sites are then added to the chain, with or without intervening nucleotides as spacers between branch sites. Orthogonal protecting or blocking groups are used on the modified nucleotides such that the protecting group that permits extension of the backbone may be removed without affecting the protecting group that permits sidechain extension.

Examples of appropriate protecting groups are also described in EPA 0317077 . Preferably dimethoxytrityl (DMT) is used as a blocking group on the sugar moiety of the nucleotide. Levulinyl or an anthraquinonyl group of the following formula: 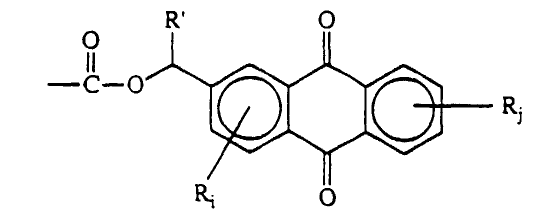 in which R' is hydrogen, aryl, or aralkyl, the Rᵢ may be the same or different and are selected from the group consisting of amino, nitro, halo, hydroxyl, lower alkyl and lower alkoxy; the Rⱼ may be the same or different and are selected from the group consisting of amino, nitro, halo, hydroxyl, lower alkyl and lower alkoxy; i is 0, 1, 2 or 3; and j is 0, 1, 2, 3 or 4, is preferably used as the blocking group on the hydroxyl moiety of the modified nucleotides. After the desired number of branch sites are incorporated, the 5' end of the molecule is extended with the S" (optional) and A segments or simply with a short S" segment (5-10 nucleotides) that provides a site for the enzymatic ligation of the A segment thereto. As indicated above, a selectable cleavage site is preferably incorporated in the extension. If the A segment is synthesized directly rather than being added by ligation, a protecting group, such as 2-methylanthraquinonyl, which may be removed selectively without adversely affecting the rest of the molecule should be used to protect the sidechain sites of the modified nucleotides.

After the 5' end of the comb body has been extended as desired, the groups protecting the hydroxyl moiety of the modified nucleotides are removed and the branching sites are extended simultaneously, preferably with the inclusion of a selectable cleavage site, so that each branch site has at least the 5-10 nucleotide extension (E) that serves as a ligation site.

The L segments (and, if not directly synthesized, the A segment, too) are then ligated to the sidechain extensions by the addition of T4 ligase and appropriate linker templates. The A and L segments and the templates may also be synthesized using available automated solid phase oligonucleotide synthesis equipment and procedures.

### Hybridization Assays

In nucleic acid hybridization assays, a large comb-type multimer of the invention is bound to the analyte nucleic acid or to a single-stranded oligonucleotide bound to the analyte. Since the multimer includes a large number (20 or more) of iterations of a sequence that are available for specific hybridization with the labeled oligonucleotide, many more label groups may be bound to the analyte than in prior procedures. The large number of label groups decreases the threshold level of detectable analyte.

The multimers may be used in essentially any of the known nucleic acid hybridization formats, such as those in which the analyte is bound directly to a solid phase or sandwich hybridizations in which the analyte is bound to an oligonucleotide that is in turn bound to a solid phase. It is particularly useful in the solution phase sandwich hybridization assay formats described in EPA0317077.

In such solution phase sandwich hybridization assays the multimer is used as follows. Single-stranded analyte nucleic acid is incubated under hybridization conditions with an excess of two single-stranded nucleic acid probe sets: (1) a set of capture probes, each having a first binding sequence complementary to the analyte and a second binding sequence that is complementary to a single-stranded oligonucleotide bound to a solid phase, and (2) a set of amplifier probes, each having a first binding sequence that is capable of specific binding to the analyte and a second binding sequence that is capable of specific binding to the A segment of the multimer. By using an amplifier probe, the multimer may be designed to be a "universal" reagent and different multimers need not be made for each analyte. The resulting product is a three component nucleic acid complex of the two probes hybridized to the analyte by their first binding sequences. The second binding sequences of the probes remain as single-stranded tails as they are not complementary to the analyte.

This complex is then added under hybridizing conditions to a solid phase having a single-stranded oligonucleotide bound to it that is complementary to the second binding sequence of the capture probe. The resulting product comprises the complex bound to the solid phase via the duplex formed by the oligonucleotide bound to the solid phase and the second binding sequence of the capture probe. The solid phase with bound complex is then separated from unbound materials.

The large comb-type amplification multimer is then added to the solid phase-analyte-probe complex under hybridization conditions to permit the multimer to hybridize to the available second binding sequence of the amplifier probe of the complex. The resulting solid phase complex is then separated from any unbound multimer by washing. The labeled oligonucleotide is then added under conditions which permit it to hybridize to the oligonucleotide units on the sidechains of the multimer. The resulting solid phase labeled nucleic acid complex is then separated from excess labeled oligonucleotide, by washing to remove unbound labeled oligonucleotide, and read.

The analyte nucleic acids may be from a variety of sources, e.g., biological fluids or solids, food stuffs, environmental materials, etc., and may be prepared for the hybridization analysis by a variety of means, e.g., proteinase K/SDS, chaotropic salts, etc. Also, it may be of advantage to decrease the average size of the analyte nucleic acids by enzymatic, physical or chemical means, e.g., restriction enzymes, sonication, chemical degradation (e.g., metal ions), etc. The fragments may be as small as 0.1 kb, usually being at least about 0.5 kb and may be 1 kb or higher. The analyte sequence is provided in single-stranded form for analysis. Where the sequence is naturally present in single-stranded form, denaturation will not be required. However, where the sequence is present in double-stranded form, the sequence will be denatured. Denaturation can be carried out by various techniques, such as alkali, generally from about 0.05 to 0.2 M hydroxide, formamide, salts, heat, or combinations thereof.

The first binding sequences of the capture probe and amplifier probe that are complementary to the analyte sequence will each be of at least 15 nucleotides, usually at least 25 nucleotides, and not more than about 5 kb, usually not more than about 1 kb, preferably not more than about 100 nucleotides. They will typically be approximately 30 nucleotides. They will normally be chosen to bind to different sequences of the analyte. The first binding sequences may be selected based on a variety of considerations. Depending upon the nature of the analyte, one may be interested in a consensus sequence, a sequence associated with polymorphisms, a particular phenotype or genotype, a particular strain, or the like.

By appropriate selection of the first binding sequences of the amplifier and capture probes they may be used to identify a specific nucleic acid molecule that includes a particular gene or other sequence that is present as part of different nucleic acid molecules. In order to discriminate the nucleic acid molecule of interest from other molecules that also contain the given sequence, one of the probes is made complementary to the given sequence while the other is made complementary to another sequence of the molecule which other sequence is unique to that molecule (i.e., is not present in the other molecules that contain the given sequence).

The second binding sequences of the capture probe and amplifier probe are selected to be complementary, respectively, to the oligonucleotide attached to the solid phase and to the A segment of the multimer and so as to not be encountered by endogenous sequences in the sample/analyte. The second binding sequence may be contiguous to the first binding sequence or be spaced therefrom by an intermediate noncomplementary sequence. The probes may include other noncomplementary sequences if desired. These noncomplementary sequences must not hinder the binding of the binding sequences or cause nonspecific binding to occur.

The capture probe and amplifier probe may be prepared by oligonucleotide synthesis procedures or by cloning, preferably the former.

It will be appreciated that the binding sequences need not have perfect complementarity to provide homoduplexes. In many situations, heteroduplexes will suffice where fewer than about 10% of the bases are mismatches, ignoring loops of five or more nucleotides. Accordingly, as used herein the term "complementary" intends a degree of complementarity sufficient to provide a stable duplex structure.

The solid phase that is used in the assay may be particulate or be the solid wall surface of any of a variety of containers, e.g., centrifugal tubes, columns, microtiter plate wells, filters, tubing, etc. When particles are used, they will preferably be of a size in the range of about 0.4 to 200 microns, more usually from about 0.8 to 4.0 microns. The particles may be any convenient material, such as latex, or glass. Microtiter plates are a preferred solid surface. The oligonucleotide that is complementary to the second binding sequence of the capture probe may be stably attached to the solid surface through functional groups by known procedures.

It will be appreciated that one can replace the second binding sequence of the capture probe and the oligonucleotide attached to the solid phase with an appropriate ligand-receptor pair that will form a stable bond joining the solid phase to the first binding sequence of the capture probe. Examples of such pairs are biotin/avidin, thyroxine/thyroxige-binding globulin, antigen/antibody, carbohydrate/lectin, and the like.

The labeled oligonucleotide will include a sequence complementary to the oligonucleotide units on the sidechains of the multimer. The labeled oligonucleotide will include one or more molecules ("labels"), which directly or indirectly provide for a detectable signal. The labels may be bound to individual members of the complementary sequence or may be present as a terminal member or terminal tail having a plurality of labels. Various means for providing labels bound to the sequence have been reported in the literature. See, for example, Leary et al., Proc Natl Acad Sci USA (1983) 80:4045; Renz and Kurz, Nucl Acids Res (1984) 12:3435; Richardson and Gumport, Nucl Acids Res (1983) 11:6167; Smith et al., Nucl Acids Res (1985) 13:2399; Meinkoth and Wahl, Anal Biochem (1984) 138:267. The labels may be bound either covalently or non-covalently to the complementary sequence. Labels which may be employed include radionuclides, fluorescers, chemiluminescers, dyes, enzymes, enzyme substrates, enzyme cofactors, enzyme inhibitors, enzyme subunits, metal ions, and the like. Illustrative specific labels include fluorescein, rhodamine, Texas red, phycoerythrin, umbelliferone, luminol, NADPH, α-β-galactosidase, horseradish peroxidase, etc.

The ratio of capture probe and amplifier probe to anticipated moles of analyte will each be at least stoichiometric and preferably in excess. This ratio is preferably at least about 1.5:1, and more preferably at least 2:1. It will normally be in the range of 2:1 to 10,000:1. Concentrations of each of the probes will generally range from about 10⁻¹⁰ to 10⁻⁶ M, with sample nucleic acid concentrations varying from 10⁻²¹ to 10⁻¹² M. The hybridization steps of the assay will generally take from about 10 minutes to 2 hours, frequently being completed in about 1 hour. Hybridization can be carried out at a mildly elevated temperature, generally in the range from about 20°C to 80°C, more usually from about 35°C to 70°C, particularly 65°C.

The hybridization reaction is usually done in an aqueous medium, particularly a buffered aqueous medium, which may include various additives. Additives which may be employed include low concentrations of detergent (0.1 to 1%), salts, e.g., sodium citrate (0.017 to 0.17 M), Ficoll, polyvinylpyrrolidine, carrier nucleic acids, carrier proteins, etc. Nonaqueous solvents may be added to the aqueous medium, such as dimethylformamide, dimethylsulfoxide, alcohols, and formamide. These other solvents will be present in amounts ranging from 2 to 50%.

The stringency of the hybridization medium may be controlled by temperature, salt concentration, solvent system, and the like. Thus, depending upon the length and nature of the sequence of interest, the stringency will be varied.

The procedure used in the separation steps of the assay will vary depending upon the nature of the solid phase. For particles, centrifugation or filtration will provide for separation of the particles, discarding the supernatant or isolating the supernatant. Where the particles are assayed, the particles will be washed thoroughly, usually from one to five times, with an appropriate buffered medium, e.g., PBS containing a detergent such as SDS. When the separation means is a wall or support, the supernatant may be isolated or discarded and the wall washed in the same manner as indicated for the particles.

Depending upon the nature of the label, various techniques can be employed for detecting the presence of the label. For fluorescers, a large number of different fluorometers are available. For chemiluminescers, luminometers or films are available. With enzymes, a fluorescent, chemiluminescent, or colored product can be provided and determined fluorometrically, luminometrically, spectrophotometrically or visually. The various labels which have been employed in immunoassays and the techniques applicable to immunoassays can be employed with the subject assays.

In a hybridization assay in which the analyte nucleic acid is bound directly to a solid phase, such as a "dot blot" assay, the multimer is hybridized directly to the bound analyte. In these instances, the A segment of the multimer is complementary to a sequence of the analyte and the oligonucleotide units on the sidechains are complementary to a labeled oligonucleotide. Unbound multimer is removed from the solid phase and the labeled oligonucleotide is then hybridized to the bound analyte-multimer complex. Excess labeled oligomer is removed and the labeled, bound complex is read.

The multimers may also be used in other assays such as direct, indirect, and sandwich immunoassays. In these instances the reagent that plays the role of the labeled antibody or other ligand that is bound directly or indirectly to the analyte has an oligonucleotide that is complementary to the A segment of the multimer bound to it rather than a label. For instance, in a sandwich immunoassay for an antigen analyte, the analyte sample is incubated with a solid phase to which is bound a first antibody to the antigen. Unbound sample is removed from the solid phase and a second antibody to the antigen and which an oligonucleotide complementary to a unit of the multimer is bound is reacted with the bound complex to form a three-membered complex. Following removal of excess second antibody the multimer is then hybridized to the complex via the oligonucleotide bound to the second antibody. Excess multimer is removed and a labeled oligonucleotide is hybridized to the other oligonucleotide units of the multimer. After removal of excess labeled oligonucleotide, the complex is read.

Kits for carrying out amplified nucleic acid hybridization assays according to the invention will comprise in packaged combination the following reagents: the multimer; an appropriate labeled oligonucleotide; a solid phase that is capable of binding to the analyte; optionally a capture probe if the assay format is One in which the analyte is bound to the solid phase through an intermediate oligonucleotide or other ligand; and optionally an amplifier probe if the assay format is one in which the multimer is not hybridized directly to the analyte. These reagents will typically be in separate containers in the kit. The kit may also include a denaturation reagent for denaturing the analyte, hybridization buffers, wash solutions, enzyme substrates, negative and positive controls and written instructions for carrying out the assay.

The following examples of the invention are offered by way of illustration and not by way of limitation.

### Example 1

This example illustrates the synthesis of a comb-type branched polynucleotide having 15 branch sites and sidechain extensions having three labeled probe binding sites. This polynucleotide was designed to be used in a solution phase hybridization as described in EPA 0317077.

All chemical syntheses of oligonucleotides were performed on an automatic DNA synthesizer (Applied Biosystems, Inc., (ABI) model 380 A/B). Phosphoramidite chemistry of the methoxy type was used except for 5'-phosphorylation which employed Phostel™ reagent (ABN). Standard ABI protocols were used except as indicated. Where it is indicated that a multiple of a cycle was used (e.g., 1.5 x cycle, 4.5 x cycle), the multiple of the standard amount of amidite recommended by ABI was employed in the specified cycle. Appended hereto are the programs for carrying out cycles 0.4, 1.5, 4.5, and CAP-PRIM as run on the Applied Biosystems Model 380 A/B DNA Synthesizer.

A comb body of the following structure was first prepared: 3'T20-X15(GTCAGTp5')15-(GTTTGTp-5')1 where X is a modified nucleotide as described previously.

The portion of the comb body through the 15 repeats is first synthesized using 40 mg thymidine controlled pore glass (CPG) (3000 Å, 3 micromoles thymidine per gram support) with a 1.5 x cycle protocol. The branching site nucleotide was of the formula: 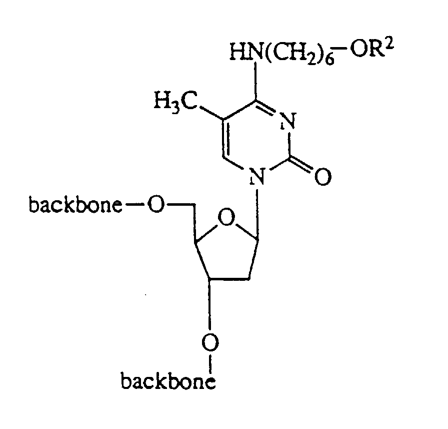 where R² represents 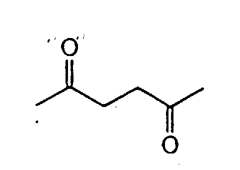 or 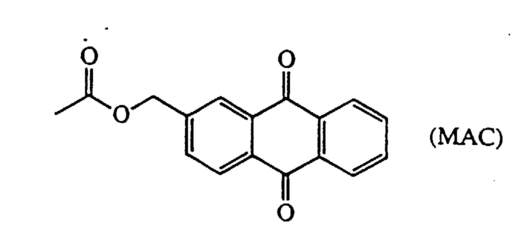 Such a nucleotide where R² represents MAC was made as follows. To a solution of N-4-(6-hydroxyhexyl)-5'-DMT-5-methyl-2'deoxycytidine (17 mmole), prepared as previously described (Horn and Urdea, NAR vol. 17:17, p. 6959-6967 (1989)), in 200 ml methylene chloride was added pyridine (40 mmole) and the mixture cooled to 0°C. A solution of 2-anthraquinonemethoxy chloroformate (MAC-C1) (20 mmole) in 200 ml of CH₂Cl₂ was added dropwise and left stirring for 10 minutes. TLC analysis (silica plates developed with 10% methanol/CH₂Cl₂) showed that the starting material had been completely consumed. The reaction mixture was diluted with 400 ml ethyl acetate and the organic phase extracted with 2 x 300 ml 5% NaHCO₃ and 80% saturated aqueous NaCl. After drying of the organic phase over Na₂SO₄ for 30 minutes followed by filtration the solvent was removed in vacuo. The product was purified by silica gel chromatography using a gradient of methanol (0-6%) in CH₂Cl₂ to give 13 g of pure product (85% yield).

A 0.1 molar solution of 2-(hydroxymethyl)anthraquinone (MAQ-OH) was prepared by dissolving 25 mmole (5.95 g) in 250 ml dioxane. The yellow solution was filtered and the solvent removed by evaporation to remove water. The residue was redissolved in 200 ml dioxane and pyridine (2 ml; 25 mmole) was added. This solution was added dropwise to a stirred solution of triphosgen (2.5 g; 25 Meq) in 50 ml CH₂Cl₂. After ended addition the mixture was stirred at 20°C for 18 hours. The mixture was diluted with 800 ml ethyl acetate and the organic phase washed with 3 x 60 ml 80% saturated aqueous NaCl solution. After drying of the organic phase over Na₂SO₄ the solvent was removed in vacuo to give a yellow solid, which was dissolved in CH₂Cl₂ (250 ml; 0.1 M). This solution was used without further purification.

The nucleoside N-4-(O-anthraquinonemethoxy carbonyl-6-oxyhexyl)-5'-DMT-5-methyl-2'-deoxycytidine (14.4 mmole) was dissolved in CH₂Cl₂ (50 ml) containing 70 mmole DiPEA. After cooling to 0°C N,N-diisopropylaminomethoxychlorophosphine was added (2.72 ml; 14 mmole). The phosphitylating agent was added in small portions until 95% of the starting material had been consumed. The reaction mixture was then diluted with ethyl acetate (300 ml), extracted with 2 x 300 ml 5% NaHCO₃ then 2 x 300 ml 80% saturated aqueous NaCl and finally dried over solid Na₂SO₄. The solvent was removed in vacuo.

The crude phosphoramidite was purified by silica gel chromatography. The purified phosphoramidite was dissolved in toluene and added with rapid stirring to 800 ml of cold hexanes (-50°C). The resulting precipitate was rapidly collected by filtration and dried in high vacuum for 18 hours to give 12.4 g (4.5 mmole, 80% yield) of a slightly yellow solid product. Deprotection of the MAC protected nucleotide is effected by treatment with sodium dithionite under neutral conditions.

For synthesis of the comb body (not including sidechains), the concentration of methylphosphoramidite monomers is 0.1 M for A, C, G and T, 0.15 M for the branching site monomer X, and 0.2 M for Phostel™ reagent. Detritylation was done with 3% trichloroacetic acid in methylene chloride using continuous flowthrough for the duration of the deprotection. At the conclusion the 5' DMT was replaced with an acetyl group.

Six base sidechain extensions of the formula 3'-GTCAGTp were synthesized at each branching monomer site as follows. The base protecting group removal (R² in the formula above) was performed manually while retaining the CPG support in the same column used for synthesizing the comb body. In the case of R² = levulinyl, a solution of 0.5 M hydrazine hydrate in pyridine/glacial acetic acid (1:1 v/v) is introduced and kept in contact with the CPG support for 90 min with renewal of the liquid every 15 min. After extensive washing with pyridine/glacial acetic acid (4:1 v/v) followed by acetonitrile, the filters in the column are replaced. In the case of R² = 2-methylanthraquinonyl a sodium dithionite solution (1 g sodium dithionite dissolved in 20 ml of 1 M trimethylammonium bicarbonate, followed by addition of 20 ml of dioxane is introduced and kept in contact with the CPG support for 90 min. After the deprotection the six base sidechain extensions were added using a 4.5 x cycle and monomer concentrations of 0.2 M.

In these syntheses the concentration of monomers is 0.2 M (including R and Phostel™ reagent). Detritylation is effected with a solution of 2.5% dichloroacetic acid in toluene/30% trichloroacetic acid in methylene chloride (1:1 v/v) using continuous flowthrough. Protecting groups were removed as follows. The phosphate protecting groups were removed from the solid-supported product fragment by treatment of the CPG with a solution of thiophenol/triethylamine/acetonitrile (1:1:2 v/v) for 1 hr at 20°C followed by washes with acetonitrile (10 x 1 ml) and methanol. The product fragment was removed from the CPG support by treatment with 0.5 ml concentrated ammonium hydroxide for 20 min and the supernatant was removed. The treatment was repeated twice for a total of one hour exposure. The combined supernatant was transferred to a screw-capped vial and heated at 60°C for 18 hr. After cooling to room temperature the solvent was removed in a Speed-Vac evaporator and the residue dissolved in 100 µl water.

5' backbone extensions (segment A), sidechain extensions and ligation template/linkers of the following structures were also made using the automatic synthesizer:

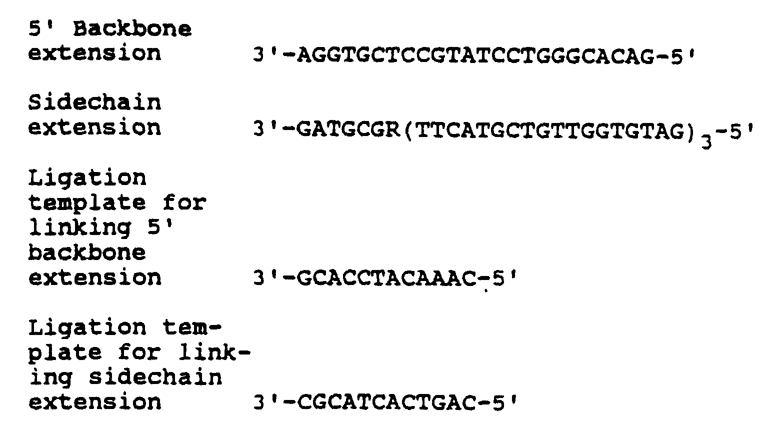

R in the sidechain extension represents the following selectable cleavage linker: 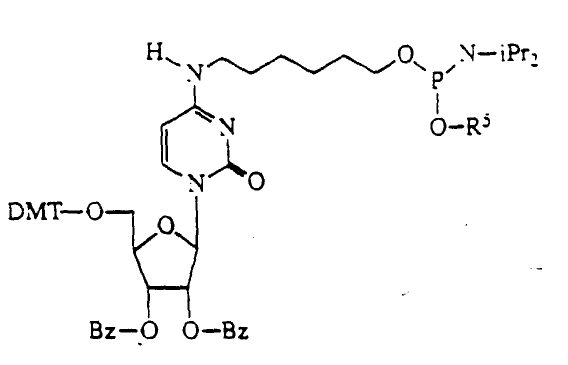 where DMT represents dimethoxytrityl, Bz represents benzoyl, R⁵ represents methyl or β-cyanoethyl, and iPr represents isopropyl.

Cleavage at the site of R is achieved with a two-step chemical procedure: (1) oxidation with aqueous NaIO₄ for 1 hr followed by (2) treatment with aqueous n-propylamine.

The crude comb body was purified by a standard polyacrylamide gel (10% with 7 M urea) method.

The 5' backbone extension and the sidechain extensions were ligated to the comb body using a standard T4 ligase protocol (Urdea (1987) Methods in Enzymol. 146:22-41), except that a longer reaction time (>8 hr), 14% polyethylene glycol, and ambient temperature are used.

After ligation and purification, a portion of the product was labeled with ³²P and subjected to the cleavage steps described above. The sample was then analyzed by PAGE to determine the number of sidechain extensions incorporated by counting the number of bands on the gel. The product was found to have a total of 24 labeled probe binding sites.

### Example 2

This example illustrates the preparation of the same multimer as made in Example 1 using a medium pore size CPG/higher loading CPG which is first adjusted to a suitable loading level. Primary synthesis was performed starting from 30 mg thymidine CPG support (1000 Å; 20 mmoles thymidine per gram support). The first 20 coupling cycles with T were performed with the 0.4 x cycle to decrease the loading to below ca. 10 mmoles per gram support. This was followed by 20 coupling cycles with T, 15 cycles with X (modified nucleotide), and finally incorporation of the sequence 3'-GTTTGTGGp using the 1.5 x cycle. The terminal 5'-DMT group was removed and the sequence capped using the CAP-PRIM cycle program on the ABI machine. The column was removed from the machine and the following manipulations performed manually. Removal of the branch-point levulinate protecting groups was performed as described above, and the resulting CPG support transferred to a new ABI column. Sidechain extension was performed as described above to incorporate the sequence 3'-GTCAGTp using the 4.5 x cycle. The protecting groups were removed as described in Example 1 (see above) and the crude product dissolved in 100 µl water.

Ligation of the A and L groups was performed as in Example 1.

### Example 3

The 24-site comb-type branched polynucleotide of Example 1 was used in a solution phase sandwich assay for N. gonorrhoeae using pilin gene-specific capture and amplifier probes and both ³²P and alkaline phosphatase-labeled probes as described in Example 5 of EPA 0317077. The two types of labels were used to assess whether use of a 24-site comb structure using alkaline phosphatase labeled probes gave any steric problems. Results were compared to those using a 5-site comb structure which had not exhibited any steric hindrance problems.

When a ³²P probe was used, the 24 site molecule gave an increase in relative output over the standard 5 site comb of 4.76 (theoretical 4.8; 195,000 ± 10,000 CPM versus 41,000 ± 1,200 CPM, respectively, at 10 attomoles). When an alkaline phosphatase labeled probe was employed, the 24 site molecule gave an increase in relative output over the standard 5 site comb of 3.94 (50.1 ± 1.7 light counts, LC, versus 12.7 ± 0.2 LC, respectively at 10 attomoles). The difference in labeling efficiency with the two types of probes indicates that the enzyme label is accommodated well in the comb structure.

Assays for the other nucleic acid analytes described in the examples of EPA 0317077 may be carried out similarly.

Modifications of the above-described modes for carrying out the invention that are obvious to those of skill in the fields of nucleic acid chemistry and nucleic acid hybridizations are intended to be within the scope of the following claims.

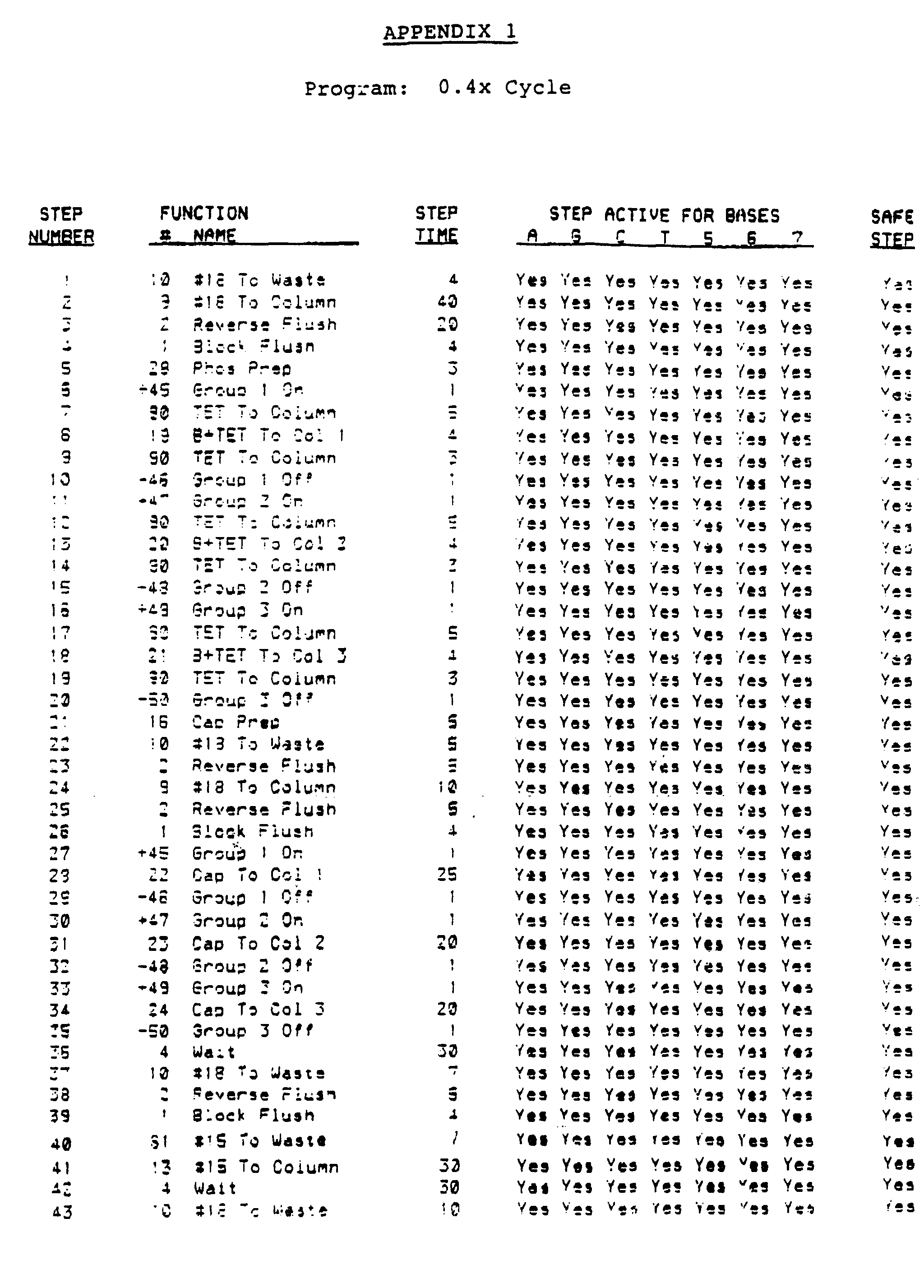

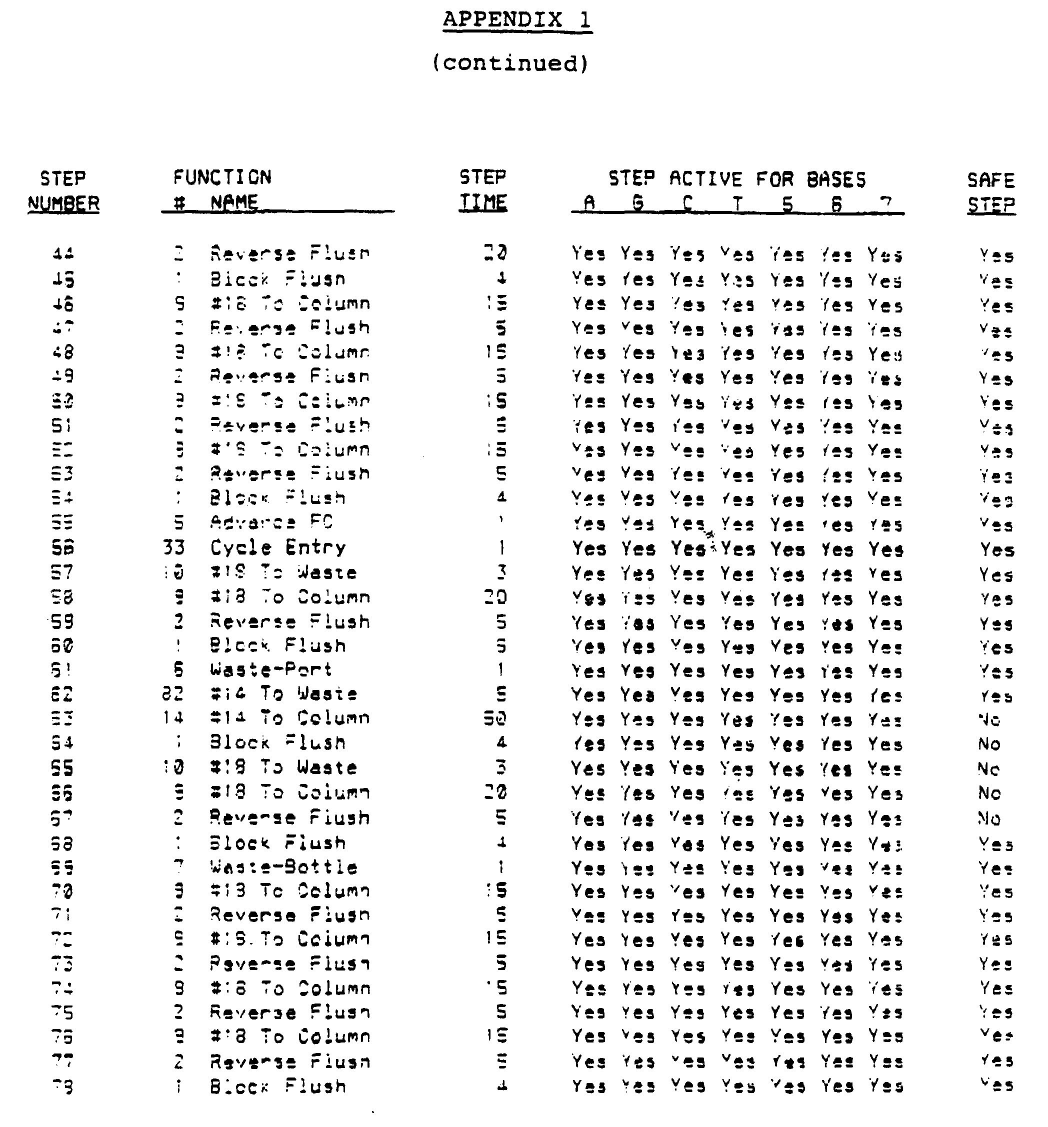

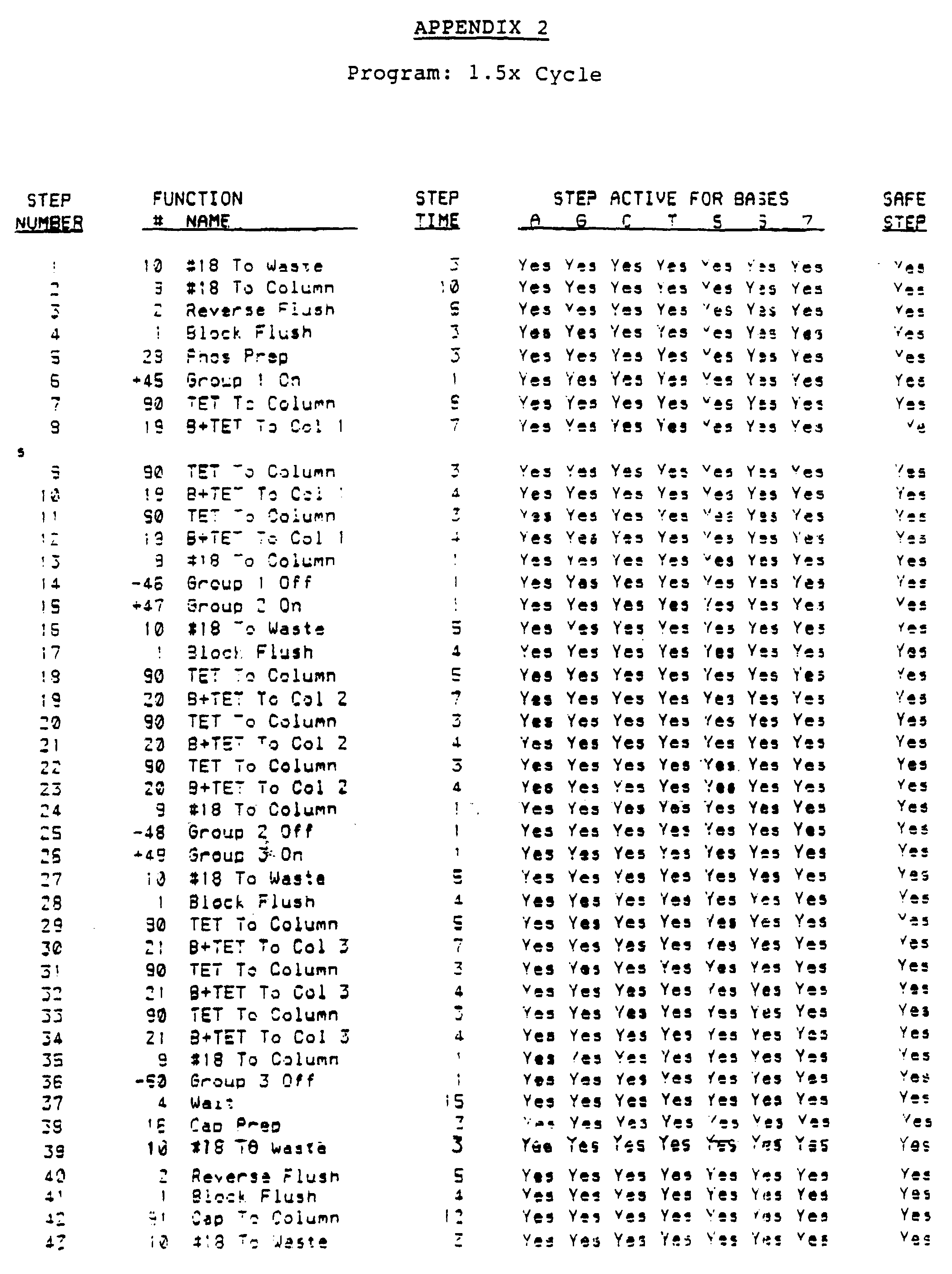

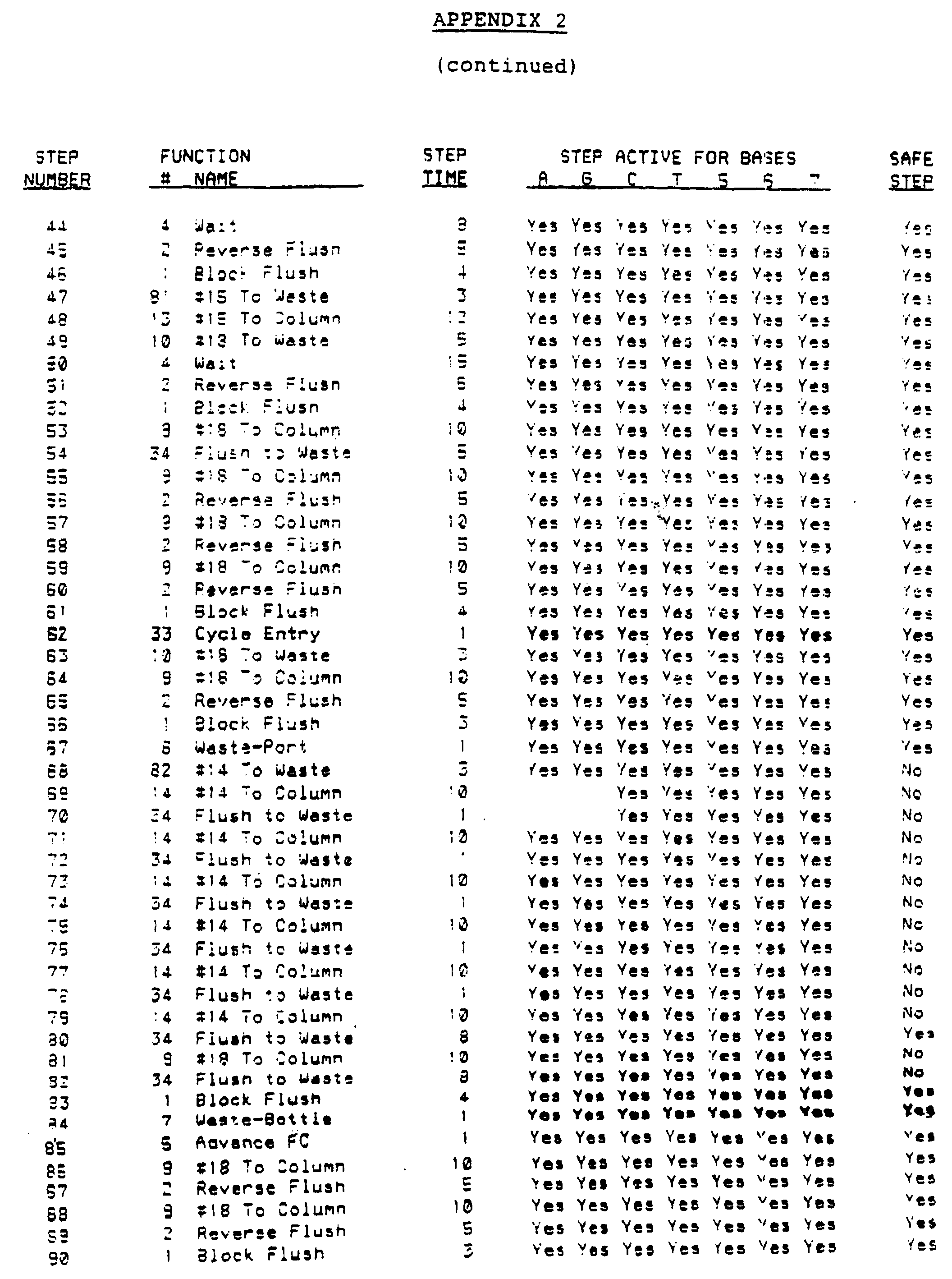

| APPENDIX 3 | | | | | | | | | | | |
|---|---|---|---|---|---|---|---|---|---|---|---|
| Program: 4.5x Cycle | | | | | | | | | | | |
| | FUNCTION | | | STEP ACTIVE FOR BASES | | | | | | | |
| STEP NUMBER | # | NAME | STEP TIME | A | G | C | T | 5 | 6 | 7 | SAFE STEP |
| 1 | 10̸ | #18 To Waste | 2 | Yes | Yes | Yes | Yes | Yes | Yes | Yes | Yes |
| 2 | 9 | #18 To Column | 15 | Yes | Yes | Yes | Yes | Yes | Yes | Yes | Yes |
| 3 | 2 | Reverse Flush | 20̸ | Yes | Yes | Yes | Yes | Yes | Yes | Yes | Yes |
| 4 | 1 | Block Flush | 4 | Yes | Yes | Yes | Yes | Yes | Yes | Yes | Yes |
| 5 | 28 | Phos Prep | 3 | Yes | Yes | Yes | Yes | Yes | Yes | Yes | Yes |
| 6 | +45 | Group 1 On | 1 | Yes | Yes | Yes | Yes | Yes | Yes | Yes | Yes |
| 7 | 90̸ | TET to column | 5 | Yes | Yes | Yes | Yes | Yes | Yes | Yes | Yes |
| 8 | 19 | B+TET To Col 1 | 5 | Yes | Yes | Yes | Yes | Yes | Yes | Yes | Yes |
| 9 | 90̸ | TET to column | 2 | Yes | Yes | Yes | Yes | Yes | Yes | Yes | Yes |
| 10̸ | -46 | Group 1 Off | 1 | Yes | Yes | Yes | Yes | Yes | Yes | Yes | Yes |
| 11 | +47 | Group 2 On | 1 | Yes | Yes | Yes | Yes | Yes | Yes | Yes | Yes |
| 12 | 90̸ | TET to column | 4 | Yes | Yes | Yes | Yes | Yes | Yes | Yes | Yes |
| 13 | 20̸ | B+TET To Col 2 | 5 | Yes | Yes | Yes | Yes | Yes | Yes | Yes | Yes |
| 14 | 90̸ | TET to column | 2 | Yes | Yes | Yes | Yes | Yes | Yes | Yes | Yes |
| 15 | -48 | Group 2 Off | 1 | Yes | Yes | Yes | Yes | Yes | Yes | Yes | Yes |
| 16 | +49 | Group 3 On | 1 | Yes | Yes | Yes | Yes | Yes | Yes | Yes | Yes |
| 17 | 90̸ | TET to column | 4 | Yes | Yes | Yes | Yes | Yes | Yes | Yes | Yes |
| 18 | 21 | B+TET To Col 3 | 5 | Yes | Yes | Yes | Yes | Yes | Yes | Yes | Yes |
| 19 | 90̸ | TET to column | 2 | Yes | Yes | Yes | Yes | Yes | Yes | Yes | Yes |
| 20̸ | -50̸ | Group 3 Off | 1 | Yes | Yes | Yes | Yes | Yes | Yes | Yes | Yes |
| 21 | 4 | Wait | 30̸ | Yes | Yes | Yes | Yes | Yes | Yes | Yes | Yes |
| 22 | +45 | Group 1 On | 1 | Yes | Yes | Yes | Yes | Yes | Yes | Yes | Yes |
| 23 | 90̸ | TET to column | 4 | Yes | Yes | Yes | Yes | Yes | Yes | Yes | Yes |
| 24 | 19 | B+TET To Col 1 | 5 | Yes | Yes | Yes | Yes | Yes | Yes | Yes | Yes |
| 25 | 90̸ | TET to column | 2 | Yes | Yes | Yes | Yes | Yes | Yes | Yes | Yes |
| 26 | -46 | Group 1 Off | 1 | Yes | Yes | Yes | Yes | Yes | Yes | Yes | Yes |
| 27 | +47 | Group 2 On | 1 | Yes | Yes | Yes | Yes | Yes | Yes | Yes | Yes |
| 28 | 90̸ | TET to column | 4 | Yes | Yes | Yes | Yes | Yes | Yes | Yes | Yes |
| 29 | 20̸ | B+TET To Col 2 | 5 | Yes | Yes | Yes | Yes | Yes | Yes | Yes | Yes |
| 30 | 90̸ | TET to column | 2 | Yes | Yes | Yes | Yes | Yes | Yes | Yes | Yes |
| 31 | -48 | Group 2 Off | 1 | Yes | Yes | Yes | Yes | Yes | Yes | Yes | Yes |
| 32 | +49 | Group 3 On | 1 | Yes | Yes | Yes | Yes | Yes | Yes | Yes | Yes |
| 33 | 90̸ | TET to column | 4 | Yes | Yes | Yes | Yes | Yes | Yes | Yes | Yes |
| 34 | 21 | B+TET To Col 3 | 5 | Yes | Yes | Yes | Yes | Yes | Yes | Yes | Yes |
| 35 | 90̸ | TET to column | 2 | Yes | Yes | Yes | Yes | Yes | Yes | Yes | Yes |
| 36 | -50̸ | Group 3 Off | 1 | Yes | Yes | Yes | Yes | Yes | Yes | Yes | Yes |
| 37 | 4 | Wait | 30 | Yes | Yes | Yes | Yes | Yes | Yes | Yes | Yes |
| 38 | +45 | Group 1 On | 1 | Yes | Yes | Yes | Yes | Yes | Yes | Yes | Yes |
| 39 | 90̸ | TET to column | 4 | Yes | Yes | Yes | Yes | Yes | Yes | Yes | Yes |
| 40̸ | 19 | B+TET To Col 1 | 5 | Yes | Yes | Yes | Yes | Yes | Yes | Yes | Yes |
| 41 | 90̸ | TET to column | 2 | Yes | Yes | Yes | Yes | Yes | Yes | Yes | Yes |
| 42 | -46 | Group 1 Off | 1 | Yes | Yes | Yes | Yes | Yes | Yes | Yes | Yes |
| 43 | +47 | Group 2 On | 1 | Yes | Yes | Yes | Yes | Yes | Yes | Yes | Yes |
| 44 | 90̸ | TET to column | 5 | Yes | Yes | Yes | Yes | Yes | Yes | Yes | Yes |
| 45 | 20̸ | B+TET To Col 2 | 5 | Yes | Yes | Yes | Yes | Yes | Yes | Yes | Yes |
| 46 | 90̸ | TET to column | 2 | Yes | Yes | Yes | Yes | Yes | Yes | Yes | Yes |
| 47 | -48 | Group 2 Off | 1 | Yes | Yes | Yes | Yes | Yes | Yes | Yes | Yes |
| 48 | +49 | Group 3 On | 1 | Yes | Yes | Yes | Yes | Yes | Yes | Yes | Yes |
| 49 | 90̸ | TET to column | 5 | Yes | Yes | Yes | Yes | Yes | Yes | Yes | Yes |
| 50̸ | 21 | B+TET To Col 3 | 5 | Yes | Yes | Yes | Yes | Yes | Yes | Yes | Yes |
| 51 | 90̸ | TET to column | 2 | Yes | Yes | Yes | Yes | Yes | Yes | Yes | Yes |
| 52 | -50̸ | Group 3 Off | 1 | Yes | Yes | Yes | Yes | Yes | Yes | Yes | Yes |
| 53 | 4 | Wait | 30̸ | Yes | Yes | Yes | Yes | Yes | Yes | Yes | Yes |
| 54 | +45 | Group 1 On | 1 | Yes | Yes | Yes | Yes | Yes | Yes | Yes | Yes |
| 55 | 90̸ | TET to column | 4 | Yes | Yes | Yes | Yes | Yes | Yes | Yes | Yes |
| 56 | 19 | B+TET To Col 1 | 5 | Yes | Yes | Yes | Yes | Yes | Yes | Yes | Yes |
| 57 | 90̸ | TET to column | 2 | Yes | Yes | Yes | Yes | Yes | Yes | Yes | Yes |
| 58 | -46 | Group 1 Off | 1 | Yes | Yes | Yes | Yes | Yes | Yes | Yes | Yes |
| 59 | +47 | Group 2 On | 1 | Yes | Yes | Yes | Yes | Yes | Yes | Yes | Yes |
| 60̸ | 90̸ | TET to column | 4 | Yes | Yes | Yes | Yes | Yes | Yes | Yes | Yes |
| 61 | 20̸ | B+TET To Col 2 | 5 | Yes | Yes | Yes | Yes | Yes | Yes | Yes | Yes |
| 62 | 90̸ | TET to column | 2 | Yes | Yes | Yes | Yes | Yes | Yes | Yes | Yes |
| 63 | -48 | Group 2 Off | 1 | Yes | Yes | Yes | Yes | Yes | Yes | Yes | Yes |
| 64 | +49 | Group 3 On | 1 | Yes | Yes | Yes | Yes | Yes | Yes | Yes | Yes |
| 65 | 90̸ | TET to column | 4 | Yes | Yes | Yes | Yes | Yes | Yes | Yes | Yes |
| 66 | 21 | B+TET To Col 3 | 5 | Yes | Yes | Yes | Yes | Yes | Yes | Yes | Yes |
| 67 | 90̸ | TET to column | 2 | Yes | Yes | Yes | Yes | Yes | Yes | Yes | Yes |
| 68 | -50̸ | Group 3 Off | 1 | Yes | Yes | Yes | Yes | Yes | Yes | Yes | Yes |
| 69 | 4 | Wait | 30̸ | Yes | Yes | Yes | Yes | Yes | Yes | Yes | Yes |
| 70̸ | +45 | Group 1 On | 1 | Yes | Yes | Yes | Yes | Yes | Yes | Yes | Yes |
| 71 | 90̸ | TET to column | 4 | Yes | Yes | Yes | Yes | Yes | Yes | Yes | Yes |
| 72 | 19 | B+TET To Col 1 | 5 | Yes | Yes | Yes | Yes | Yes | Yes | Yes | Yes |
| 73 | 90̸ | TET to column | 2 | Yes | Yes | Yes | Yes | Yes | Yes | Yes | Yes |
| 74 | -46 | Group 1 Off | 1 | Yes | Yes | Yes | Yes | Yes | Yes | Yes | Yes |
| 75 | +47 | Group 2 On | 1 | Yes | Yes | Yes | Yes | Yes | Yes | Yes | Yes |
| 76 | 90̸ | TET to column | 4 | Yes | Yes | Yes | Yes | Yes | Yes | Yes | Yes |
| 77 | 20̸ | B+TET To Col 2 | 5 | Yes | Yes | Yes | Yes | Yes | Yes | Yes | Yes |
| 78 | 90̸ | TET to column | 2 | Yes | Yes | Yes | Yes | Yes | Yes | Yes | Yes |
| 79 | -48 | Group 2 Off | 1 | Yes | Yes | Yes | Yes | Yes | Yes | Yes | Yes |
| 80̸ | +49 | Group 3 On | 1 | Yes | Yes | Yes | Yes | Yes | Yes | Yes | Yes |
| 81 | 90̸ | TET to column | 4 | Yes | Yes | Yes | Yes | Yes | Yes | Yes | Yes |
| 82 | 21 | B+TET To Col 3 | 5 | Yes | Yes | Yes | Yes | Yes | Yes | Yes | Yes |
| 83 | 90̸ | TET to column | 2 | Yes | Yes | Yes | Yes | Yes | Yes | Yes | Yes |
| 84 | -50̸ | Group 3 Off | 1 | Yes | Yes | Yes | Yes | Yes | Yes | Yes | Yes |
| 85 | 4 | Wait | 30̸ | Yes | Yes | Yes | Yes | Yes | Yes | Yes | Yes |
| 86 | +45 | Group 1 On | 1 | Yes | Yes | Yes | Yes | Yes | Yes | Yes | Yes |
| 87 | 90̸ | TET to column | 4 | Yes | Yes | Yes | Yes | Yes | Yes | Yes | Yes |
| 88 | 19 | B+TET To Col 1 | 5 | Yes | Yes | Yes | Yes | Yes | Yes | Yes | Yes |
| 89 | 90̸ | TET to column | 2 | Yes | Yes | Yes | Yes | Yes | Yes | Yes | Yes |
| 90̸ | -46 | Group 1 Off | 1 | Yes | Yes | Yes | Yes | Yes | Yes | Yes | Yes |
| 91 | +47 | Group 2 On | 1 | Yes | Yes | Yes | Yes | Yes | Yes | Yes | Yes |
| 92 | 90̸ | TET to column | 4 | Yes | Yes | Yes | Yes | Yes | Yes | Yes | Yes |
| 93 | 20̸ | B+TET To Col 2 | 5 | Yes | Yes | Yes | Yes | Yes | Yes | Yes | Yes |
| 94 | 90̸ | TET to column | 2 | Yes | Yes | Yes | Yes | Yes | Yes | Yes | Yes |
| 95 | -48 | Group 2 Off | 1 | Yes | Yes | Yes | Yes | Yes | Yes | Yes | Yes |
| 96 | +49 | Group 3 On | 1 | Yes | Yes | Yes | Yes | Yes | Yes | Yes | Yes |
| 97 | 90̸ | TET to column | 4 | Yes | Yes | Yes | Yes | Yes | Yes | Yes | Yes |
| 98 | 21 | B+TET To Col 3 | 5 | Yes | Yes | Yes | Yes | Yes | Yes | Yes | Yes |
| 99 | 90̸ | TET to column | 2 | Yes | Yes | Yes | Yes | Yes | Yes | Yes | Yes |
| 10̸0̸ | -50̸ | Group 3 Off | 1 | Yes | Yes | Yes | Yes | Yes | Yes | Yes | Yes |
| 10̸1 | 4 | Wait | 30̸ | Yes | Yes | Yes | Yes | Yes | Yes | Yes | Yes |
| 10̸2 | +45 | Group 1 On | 1 | Yes | Yes | Yes | Yes | Yes | Yes | Yes | Yes |
| 10̸3 | 90̸ | TET to column | 4 | Yes | Yes | Yes | Yes | Yes | Yes | Yes | Yes |
| 10̸4 | 19 | B+TET To Col 1 | 5 | Yes | Yes | Yes | Yes | Yes | Yes | Yes | Yes |
| 10̸5 | 90̸ | TET to column | 2 | Yes | Yes | Yes | Yes | Yes | Yes | Yes | Yes |
| 10̸6 | -46 | Group 1 Off | 1 | Yes | Yes | Yes | Yes | Yes | Yes | Yes | Yes |
| 10̸7 | +47 | Group 2 On | 1 | Yes | Yes | Yes | Yes | Yes | Yes | Yes | Yes |
| 10̸8 | 90̸ | TET to column | 4 | Yes | Yes | Yes | Yes | Yes | Yes | Yes | Yes |
| 10̸9 | 20̸ | B+TET To Col 2 | 5 | Yes | Yes | Yes | Yes | Yes | Yes | Yes | Yes |
| 110̸ | 90̸ | TET to column | 2 | Yes | Yes | Yes | Yes | Yes | Yes | Yes | Yes |
| 111 | -48 | Group 2 Off | 1 | Yes | Yes | Yes | Yes | Yes | Yes | Yes | Yes |
| 112 | +49 | Group 3 On | 1 | Yes | Yes | Yes | Yes | Yes | Yes | Yes | Yes |
| 113 | 90̸ | TET to column | 4 | Yes | Yes | Yes | Yes | Yes | Yes | Yes | Yes |
| 114 | 21 | B+TET To Col 3 | 5 | Yes | Yes | Yes | Yes | Yes | Yes | Yes | Yes |
| 115 | 90̸ | TET to column | 2 | Yes | Yes | Yes | Yes | Yes | Yes | Yes | Yes |
| 116 | -50̸ | Group 3 Off | 1 | Yes | Yes | Yes | Yes | Yes | Yes | Yes | Yes |
| 117 | 4 | Wait | 30̸ | Yes | Yes | Yes | Yes | Yes | Yes | Yes | Yes |
| 118 | +45 | Group 1 On | 1 | Yes | Yes | Yes | Yes | Yes | Yes | Yes | Yes |
| 119 | 90̸ | TET to column | 4 | Yes | Yes | Yes | Yes | Yes | Yes | Yes | Yes |
| 120̸ | 19 | B+TET To Col 1 | 5 | Yes | Yes | Yes | Yes | Yes | Yes | Yes | Yes |
| 121 | 90̸ | TET to column | 2 | Yes | Yes | Yes | Yes | Yes | Yes | Yes | Yes |
| 122 | -46 | Group 1 Off | 1 | Yes | Yes | Yes | Yes | Yes | Yes | Yes | Yes |
| 123 | +47 | Group 2 On | 1 | Yes | Yes | Yes | Yes | Yes | Yes | Yes | Yes |
| 124 | 90̸ | TET to column | 4 | Yes | Yes | Yes | Yes | Yes | Yes | Yes | Yes |
| 125 | 20̸ | B+TET To Col 2 | 5 | Yes | Yes | Yes | Yes | Yes | Yes | Yes | Yes |
| 126 | 90̸ | TET to column | 2 | Yes | Yes | Yes | Yes | Yes | Yes | Yes | Yes |
| 127 | -48 | Group 2 Off | 1 | Yes | Yes | Yes | Yes | Yes | Yes | Yes | Yes |
| 128 | +49 | Group 3 On | 1 | Yes | Yes | Yes | Yes | Yes | Yes | Yes | Yes |
| 129 | 90̸ | TET to column | 4 | Yes | Yes | Yes | Yes | Yes | Yes | Yes | Yes |
| 130̸ | 21 | B+TET To Col 3 | 5 | Yes | Yes | Yes | Yes | Yes | Yes | Yes | Yes |
| 131 | 90̸ | TET to column | 2 | Yes | Yes | Yes | Yes | Yes | Yes | Yes | Yes |
| 132 | -50̸ | Group 3 Off | 1 | Yes | Yes | Yes | Yes | Yes | Yes | Yes | Yes |
| 133 | 4 | Wait | 30̸ | Yes | Yes | Yes | Yes | Yes | Yes | Yes | Yes |
| 134 | +45 | Group 1 On | 1 | Yes | Yes | Yes | Yes | Yes | Yes | Yes | Yes |
| 135 | 90̸ | TET to column | 4 | Yes | Yes | Yes | Yes | Yes | Yes | Yes | Yes |
| 136 | 19 | B+TET To Col 1 | 5 | Yes | Yes | Yes | Yes | Yes | Yes | Yes | Yes |
| 137 | 90̸ | TET to column | 2 | Yes | Yes | Yes | Yes | Yes | Yes | Yes | Yes |
| 138 | -46 | Group 1 Off | 1 | Yes | Yes | Yes | Yes | Yes | Yes | Yes | Yes |
| 139 | +47 | Group 2 On | 1 | Yea | Yes | Yes | Yes | Yes | Yes | Yes | Yes |
| 140̸ | 90̸ | TET to column | 4 | Yes | Yes | Yes | Yes | Yes | Yes | Yes | Yes |
| 141 | 20̸ | B+TET To Col 2 | 5 | Yes | Yes | Yes | Yes | Yes | Yes | Yes | Yes |
| 142 | 90̸ | TET to column | 2 | Yes | Yes | Yes | Yes | Yes | Yes | Yes | Yes |
| 143 | -48 | Group 2 Off | 1 | Yes | Yes | Yes | Yes | Yes | Yes | Yes | Yes |
| 144 | +49 | Group 3 On | 1 | Yes | Yes | Yes | Yes | Yes | Yes | Yes | Yes |
| 145 | 90̸ | TET to column | 4 | Yes | Yes | Yes | Yes | Yes | Yes | Yes | Yes |
| 146 | 21 | B+TET To Col 3 | 5 | Yes | Yes | Yes | Yes | Yes | Yes | Yes | Yes |
| 147 | 90̸ | TET to column | 2 | Yes | Yes | Yes | Yes | Yes | Yes | Yes | Yes |
| 148 | -50̸ | Group 3 Off | 1 | Yes | Yes | Yes | Yes | Yes | Yes | Yes | Yes |
| 149 | 4 | Wait | 30̸ | Yes | Yes | Yes | Yes | Yes | Yes | Yes | Yes |
| 150̸ | 16 | Cap Prep | 10̸ | Yes | Yes | Yes | Yes | Yes | Yes | Yes | Yes |
| 151 | 10̸ | #18 To Waste | 4 | Yes | Yes | Yes | Yes | Yes | Yes | Yes | Yes |
| 152 | 2 | Reverse Flush | 10̸ | Yes | Yes | Yes | Yes | Yes | Yes | Yes | Yes |
| 153 | 1 | Block Flush | 4 | Yes | Yes | Yes | Yes | Yes | Yes | Yes | Yes |
| 154 | +45 | Group 1 On | 1 | Yes | Yes | Yes | Yes | Yes | Yes | Yes | Yes |
| 155 | 22 | Cap To Col 1 | 26 | Yes | Yes | Yes | Yes | Yes | Yes | Yes | Yes |
| 156 | -46 | Group 1 Off | 1 | Yes | Yes | Yes | Yes | Yes | Yes | Yes | Yes |
| 157 | +47 | Group 2 On | 1 | Yes | Yes | Yes | Yes | Yes | Yes | Yes | Yes |
| 158 | 23 | Cap To Col 2 | 21 | Yes | Yes | Yes | Yes | Yes | Yes | Yes | Yes |
| 159 | -48 | Group 2 Off | 1 | Yes | Yes | Yes | Yes | Yes | Yes | Yes | Yes |
| 160̸ | +49 | Group 3 On | 1 | Yes | Yes | Yes | Yes | Yes | Yes | Yes | Yes |
| 161 | 24 | Cap To Col 3 | 21 | Yes | Yes | Yes | Yes | Yes | Yes | Yes | Yes |
| 162 | -50̸ | Group 3 Off | 1 | Yes | Yes | Yes | Yes | Yes | Yes | Yes | Yes |
| 163 | 4 | Wait | 30̸ | Yes | Yes | Yes | Yes | Yes | Yes | Yes | Yes |
| 164 | 10̸ | #18 To Waste | 3 | Yes | Yes | Yes | Yes | Yes | Yes | Yes | Yes |
| 165 | 2 | Reverse Flush | 10̸ | Yes | Yes | Yes | Yes | Yes | Yes | Yes | Yes |
| 166 | 1 | Block Flush | 4 | Yes | Yes | Yes | Yes | Yes | Yes | Yes | Yes |
| 167 | 81 | #15 To Waste | 4 | Yes | Yes | Yes | Yes | Yes | Yes | Yes | Yes |
| 168 | 13 | #15 To Column | 16 | Yes | Yes | Yes | Yes | Yes | Yes | Yes | Yes |
| 169 | 4 | Wait | 30̸ | Yes | Yes | Yes | Yes | Yes | Yes | Yes | Yes |
| 170̸ | 10̸ | #18 To Waste | 3 | Yes | Yes | Yes | Yes | Yes | Yes | Yes | Yes |
| 171 | 2 | Reverse Flush | 20̸ | Yes | Yes | Yes | Yes | Yes | Yes | Yes | Yes |
| 172 | 1 | Block Flush | 4 | Yes | Yes | Yes | Yes | Yes | Yes | Yes | Yes |
| 173 | 9 | #18 To Column | 15 | Yes | Yes | Yes | Yes | Yes | Yes | Yes | Yes |
| 174 | 2 | Reverse Flush | 10̸ | Yes | Yes | Yes | Yes | Yes | Yes | Yes | Yes |
| 175 | 9 | #18 To Column | 15 | Yes | Yes | Yes | Yes | Yes | Yes | Yes | Yes |
| 176 | 2 | Reverse Flush | 10̸ | Yes | Yes | Yes | Yes | Yes | Yes | Yes | Yes |
| 177 | 9 | #18 To Column | 15 | Yes | Yes | Yes | Yes | Yes | Yes | Yes | Yes |
| 178 | 2 | Reverse Flush | 9 | Yes | Yes | Yes | Yes | Yes | Yes | Yes | Yes |
| 179 | 9 | #18 To Column | 15 | Yes | Yes | Yes | Yes | Yes | Yes | Yes | Yes |
| 180̸ | 2 | Reverse Flush | 9 | Yes | Yes | Yes | Yes | Yes | Yes | Yes | Yes |
| 181 | 1 | Block Flush | 4 | Yes | Yes | Yes | Yes | Yes | Yes | Yes | Yes |
| 182 | 33 | Cycle Entry | 1 | Yes | Yes | Yes | Yes | Yes | Yes | Yes | Yes |
| 183 | 10̸ | #18 To Waste | 2 | Yes | Yes | Yes | Yes | Yes | Yes | Yes | Yes |
| 184 | 9 | #18 To Column | 15 | Yes | Yes | Yes | Yes | Yes | Yes | Yes | Yes |
| 185 | 2 | Reverse Flush | 9 | Yes | Yes | Yes | Yes | Yes | Yes | Yes | Yes |
| 186 | 1 | Block Flush | 4 | Yes | Yes | Yes | Yes | Yes | Yes | Yes | Yes |
| 187 | 6 | Waste-Port | 1 | Yes | Yes | Yes | Yes | Yes | Yes | Yes | Yes |
| 188 | 82 | #14 To Waste | 3 | Yes | Yes | Yes | Yes | Yes | Yes | Yes | Yes |
| 189 | 14 | #14 To Column | 65 | Yes | Yes | Yes | Yes | Yes | Yes | Yes | No |
| 190̸ | 1 | Block Flush | 6 | Yes | Yes | Yes | Yes | Yes | Yes | Yes | Yes |
| 191 | 7 | Waste-Bottle | 1 | Yes | Yes | Yes | Yes | Yees | Yes | Yes | Yes |
| 192 | 5 | Advance FC | 1 | Yes | Yes | Yes | Yes | Yes | Yes | Yes | Yes |
| 193 | 10̸ | #18 To Waste | 3 | Yes | Yes | Yes | Yes | Yes | Yes | Yes | No |
| 194 | 9 | #18 To Column | 15 | Yes | Yes | Yes | Yes | Yes | Yes | Yes | No |
| 195 | 2 | Reverse Flush | 10̸ | Yes | Yes | Yes | Yes | Yes | Yes | Yes | No |
| 196 | 9 | #18 To Column | 15 | Yes | Yes | Yes | Yes | Yes | Yes | Yes | Yes |
| 197 | 2 | Reverse Flush | 10̸ | Yes | Yes | Yes | Yes | Yes | Yes | Yes | Yes |
| 198 | 9 | #18 To Column | 15 | Yes | Yes | Yes | Yes | Yes | Yes | Yes | Yes |
| 199 | 2 | Reverse Flush | 10̸ | Yes | Yes | Yes | Yes | Yes | Yes | Yes | Yes |
| 20̸0̸ | 1 | Block Flush | 4 | Yes | Yes | Yes | Yes | Yes | Yes | Yes | Yes |

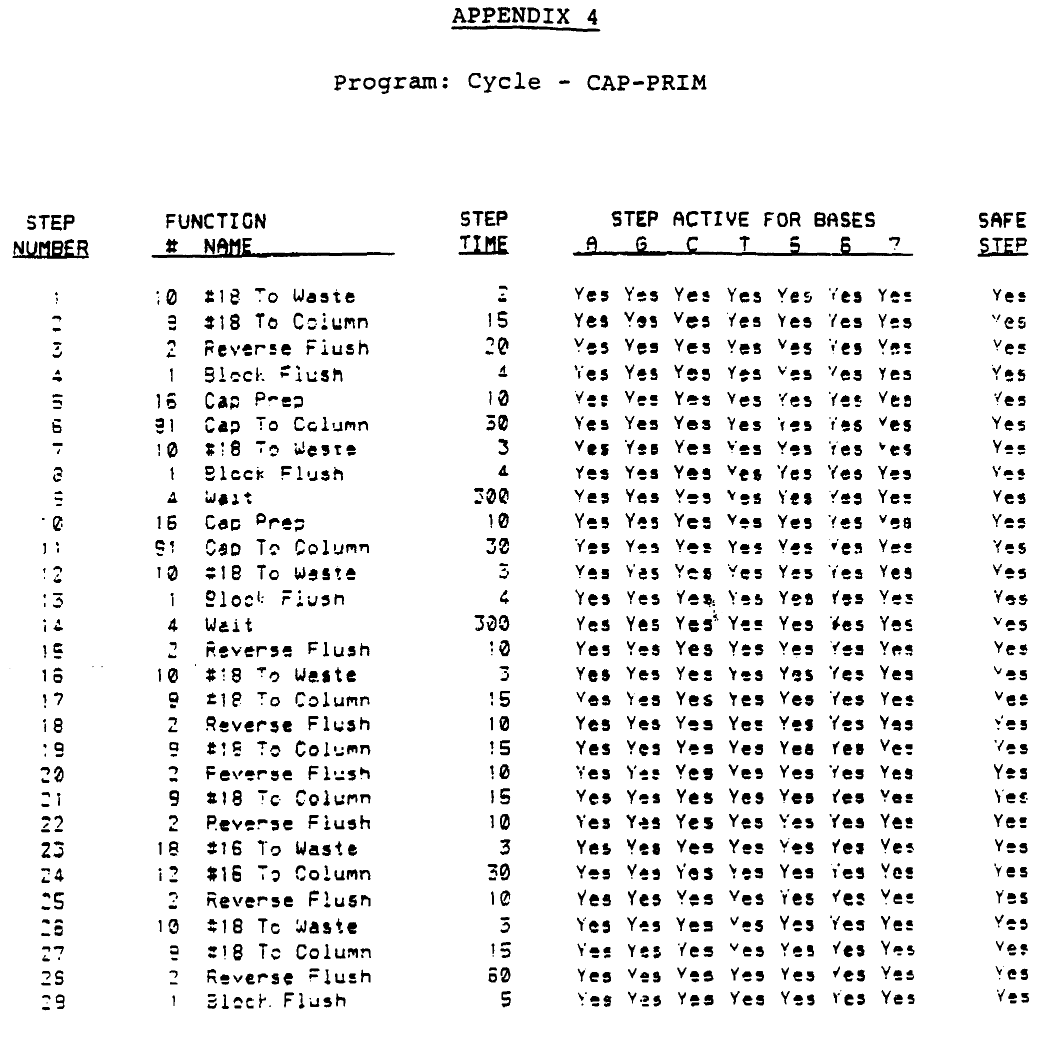

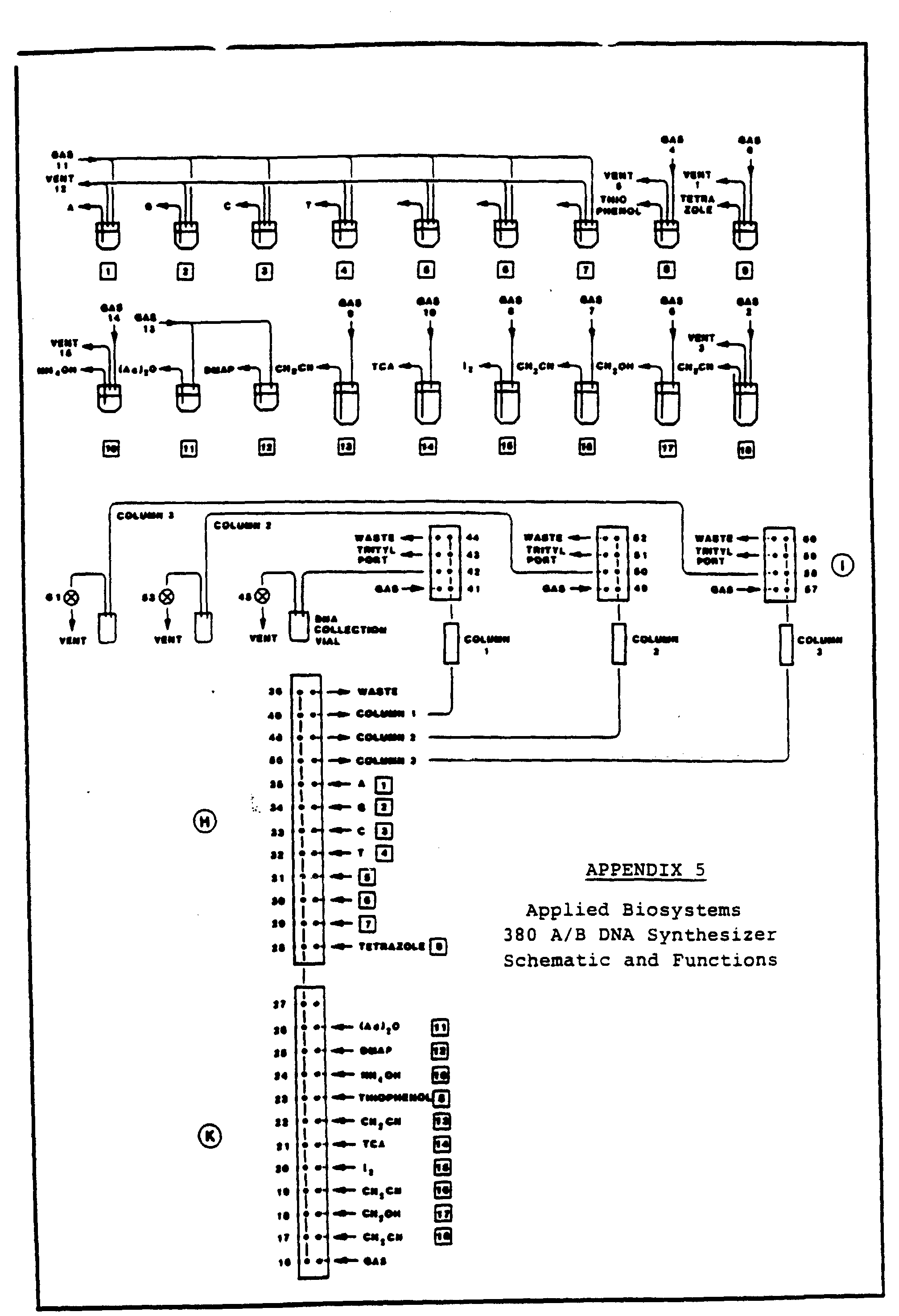

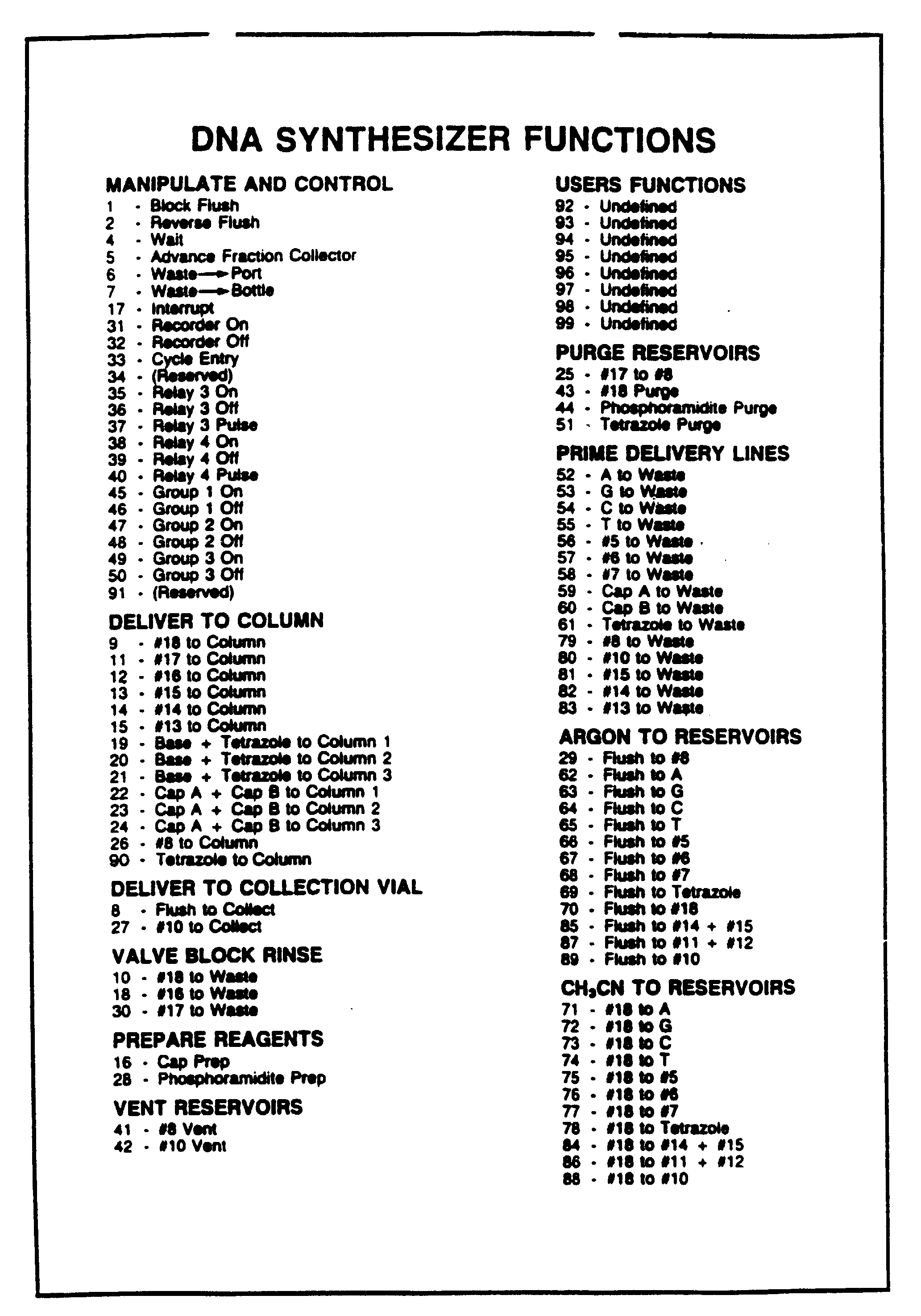

## Claims

1. A large comb-type branched polynucleotide comprising:
(a) a polynucleotide backbone having:
(i) at least 15 multifunctional nucleotides, each of which defines a sidechain site and
(ii) a first single-stranded oligonucleotide unit that is capable of binding specifically to a first single-stranded polynucleotide sequence of interest; and
(b) pendant polynucleotide sidechains extending from said multifunctional nucleotides, at least 70% of which comprise iterations of a second single-stranded oligonucleotide unit that is capable of binding specifically to a second single-stranded polynucleotide sequence of interest, the total number of iterations in all sidechains being at least 20.

2. The branched polynucleotide of claim 1 wherein the first single-stranded polynucleotide sequence of interest is analyte nucleic acid or a polynucleotide bound to analyte nucleic acid and the second single-stranded polynucleotide sequence of interest is a labeled polynucleotide.

3. The branched polynucleotide of claim 2 wherein there are 15 to 50 multifunctional nucleotides.

4. The branched polynucleotide of claim 2 wherein the multifunctional nucleotide is of the formula 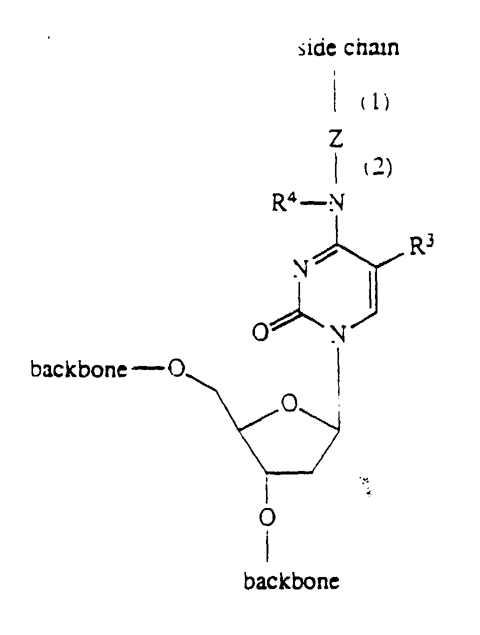 where R³ is hydrogen, methyl, I , Br or F, R⁴ is hydrogen or methyl, Z is selected from the group consisting of 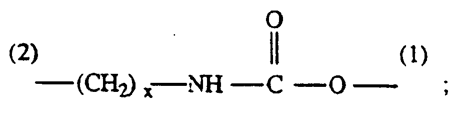 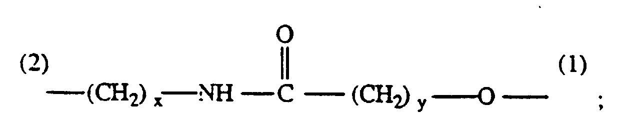 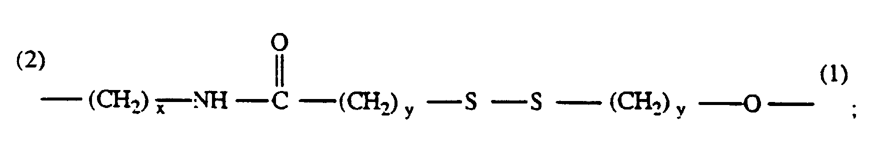 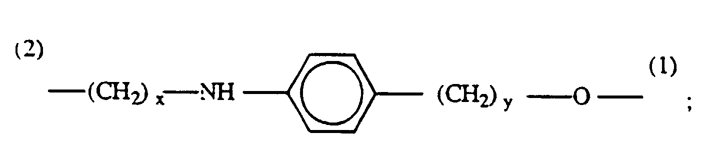
⁽²⁾―(CH₂―CH₂―O)ₓ―⁽¹⁾;
and
⁽²⁾―(CH₂)ₓ―O―⁽¹⁾,
wherein x and y may be the same or different and are integers in the range of 1 to 8 inclusive.

5. The branched polynucleotide of claim 2 wherein the first and second single-stranded oligonucleotide units are each 15 to 50 nucleotides long.

6. The branched polynucleotide of claim 2 wherein the number of iterations in each sidechain is 2 to 10.

7. The branched polynucleotide of claim 1 wherein the branched polynucleotide is of the formula 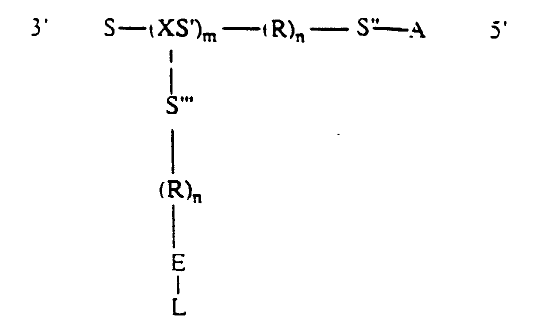 where S is a first spacer segment of at least about 15 nucleotides, X is a multifunctional nucleotide that provides a branch site, S' is a branching site spacer segment of 0 to 15 nucleotides, m is an integer equal to or greater than 15, R is a cleavable linker molecule, n is 0 or 1, S" is a second spacer segment of about 0 to 10 nucleotides, A is a nucleotide segment that is capable of hybridizing specifically to analyte nucleic acid or nucleic acid bound to analyte, S''' is a third spacer segment of 0 to 10 nucleotides, E is an oligonucleotide extension of 5 to 10 nucleotides, and L is a segment containing 2 to 10 iterations, of a nucleotide sequence that is capable of hybridizing specifically to a labeled oligonucleotide probe.

8. The branched polynucleotide of claim 7 wherein S is 15 to 50 nucleotides in length, X is of the formula 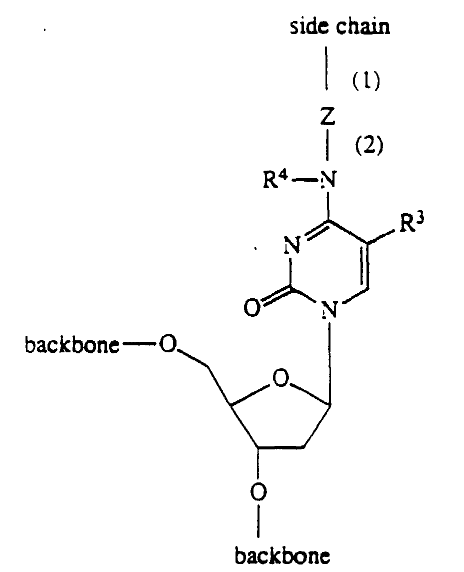 where R³ is hydrogen, methyl, I , Br or F, R⁴ is hydrogen or methyl, Z is selected from the group consisting of 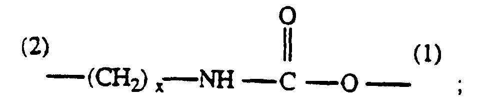 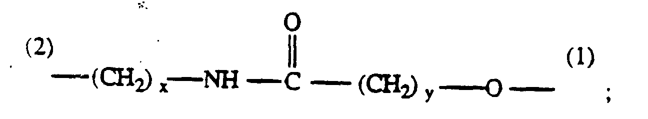 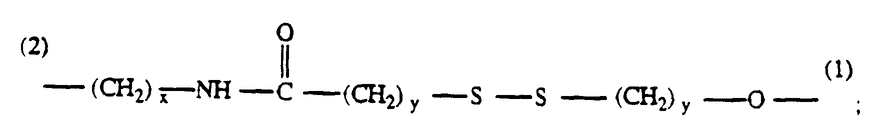 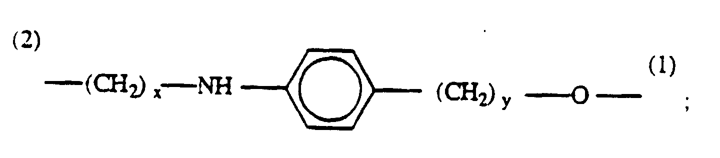
⁽²⁾―(CH₂―CH₂―O)ₓ―⁽¹⁾ ;
and
⁽²⁾―(CH₂)ₓ―O―⁽¹⁾ ,
wherein x and y may be the same or different and are integers in the range of 1 to 8 inclusive, S" is 5 to 10 nucleotides in length, and the number of iterations in L is 3 to 6.

9. The branched polynucleotide of claim 8 wherein S is polyT.

10. The branched polynucleotide of claim 8 wherein n is 1.

11. The branched polynucleotide of claim 10 wherein the cleavable linker molecule is of the formula 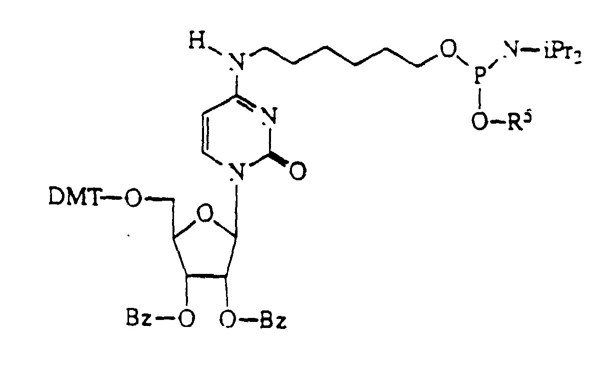 where DMT represents dimethoxytrityl, Bz represents benzoyl, R⁵ represents methyl or β-cyanoethyl, and iPr represents isopropyl.

12. A process for making a large comb-type branched polynucleotide of Claim 1, useful as an amplification multimer in a nucleic acid hybridization assay, comprising:
(a) synthesizing a single-stranded polynucleotide backbone comprising:
(i) at least 15 multifunctional nucleotides, each of which has a protected functional group that serves as a site for sidechain nucleotide extension and
(ii) a first ligation site segment;
(b) deprotecting said functional groups;
(c) extending each of said sites at least about five nucleotides to provide second ligation site segments;
(d) ligating a first single-stranded oligonucleotide unit to the first ligation site, said first single-stranded oligonucleotide unit being capable of binding to a first single-stranded nucleic acid sequence of interest; and
(e) ligating second single-stranded oligonucleotide units to the second ligation site segments, said second single-stranded oligonucleotide units comprising iterations of a sequence that is capable of binding to a second single-stranded oligonucleotide of interest.

13. The process of claim 12 wherein said synthesis and extension are carried out via a solid-phase process in which the 3' end of the backbone is affixed to the solid phase and the backbone includes a 3' leader sequence of at least 15 nucleotides.

14. The process of claim 13 wherein the solid phase is controlled pore glass of at least 2000 A pore size.

15. The process of claim 14 wherein said site for nucleotide extension is extended 5-10 nucleotides.

16. The process of claim 13 wherein the multifunctional nucleotide is of the formula 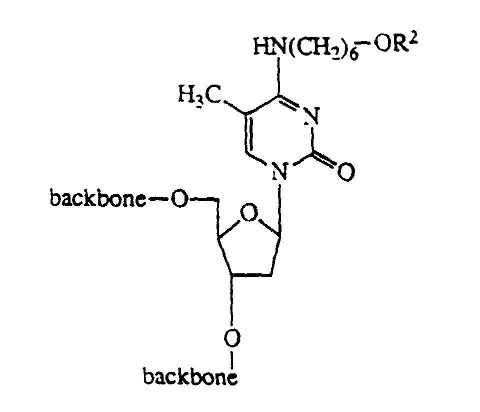 where R² represents 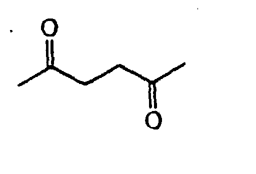 or

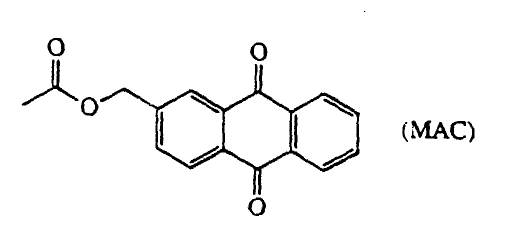

17. A process for making a large comb-type branched polynucleotide of Claim 1, useful as an amplification multimer in a nucleic acid hybridization assay, comprising:
(a) synthesizing a single-stranded polynucleotide backbone comprising:
(i) at least 15 multifunctional nucleotides, each of which has a protected functional group that serves as a site for sidechain nucleotide extension and
(ii) a first single-stranded oligonucleotide unit that is capable of binding specifically to a first single-stranded polynucleotide sequence of interest;
(b) deprotecting said functional groups;
(c) extending each of said sites at least about five nucleotides to provide ligation site segments; and
(d) ligating second single-stranded oligonucleotide units to the ligation site segments said second single-stranded oligonucleotide units comprising iterations of a sequence that is capable of binding to a second single-stranded oligonucleotide of interest.

18. The process of claim 17 wherein the multifunctional nucleotide is of the formula 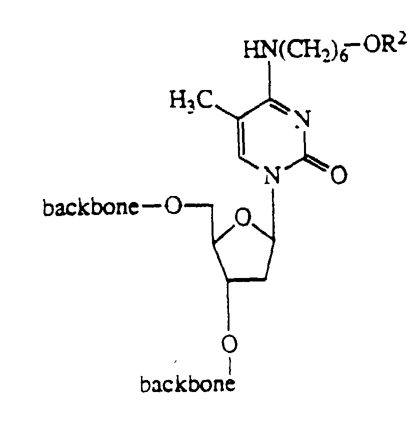 where R² represents 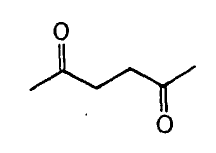 or

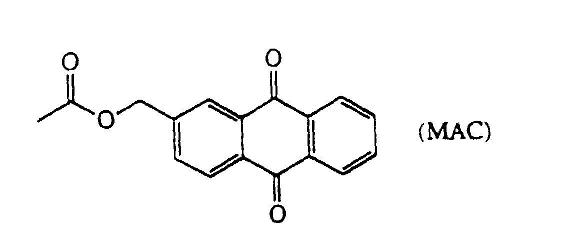

19. The process of claim 18 wherein said synthesis and extension are carried out via a solid-phase process in which the 3' end of the backbone is affixed to the solid phase and the backbone includes a 3' spacer sequence of at least 15 nucleotides.

20. The process of claim 18 wherein the solid phase is controlled pore glass of at least 2000 Å pore size.

21. The process of claim 18 wherein said site for nucleotide extension is extended 5-10 nucleotides.

22. A nucleic acid hybridization assay wherein analyte nucleic acid is hybridized to a labeled nucleic acid probe, which comprises hybridizing the branched polynucleotide of claim 2 directly or indirectly to the analyte via the first single-stranded oligonucleotide unit and hybridizing the labeled nucleic acid probe to the branched polynucleotide via the second single-stranded oligonucleotide unit.

23. A nucleic acid hybridization assay wherein analyte nucleic acid is hybridized to a labeled nucleic acid probe, which comprises hybridizing the branched polynucleotide of claim 7 directly or indirectly to the analyte via the first single-stranded oligonucleotide unit and hybridizing the labeled nucleic acid probe to the branched polynucleotide via the second single-stranded oligonucleotide unit.

24. A nucleic acid hybridization assay wherein:
(a) the branched polynucleotide of claim 2 is hybridized via the first oligonucleotide unit to a single-stranded analyte nucleic acid bound to a solid phase or to a single-stranded oligonucleotide bound to the analyte;
(b) unbound branched polynucleotide is removed;
(c) single-stranded labeled oligonucleotide is hybridized to the branched polynucleotide via the second oligonucleotide units;
(d) unbound labeled oligonucleotide is removed; and
(e) the presence of label bound to the branched polynucleotide is detected.

25. A nucleic acid hybridization assay wherein:
(a) the branched polynucleotide of claim 7 is hybridized via the first oligonucleotide unit to single-stranded analyte nucleic acid bound to a solid phase or to a single-stranded oligonucleotide bound to the analyte;
(b) unbound branched polynucleotide is removed;
(c) single-stranded labeled oligonucleotide is hybridized to the branched polynucleotide via the second oligonucleotide units;
(d) unbound labeled oligonucleotide is removed; and
(e) the presence of label bound to the branched polynucleotide is detected.

## Patentansprüche

1. Großes kammartig verzweigtes Polynukleotid, umfassend:
(a) ein Polynukleotid-Rückgrat mit:
(i) mindestens 15 multifunktionellen Nukleotiden, von denen jedes eine Seitenkettenstelle definiert, und
(ii) einer ersten einzelsträngigen Oligonukleotideinheit, welche an eine erste einzelsträngige Polynukleotidsequenz von Interesse spezifisch binden kann, und
(b) daran hängend Polynukleotid-Seitenketten, welche sich von den genannten multifunktionellen Nukleotiden erstrecken, und von denen mindestens 70% Wiederholungen einer zweiten einzelsträngigen Oligonukleotideinheit, welche eine zweite einzelsträngige Polynukleotidsequenz von Interesse spezifisch binden kann, umfassen, wobei die Gesamtzahl der Wiederholungen in allen Seitenketten mindestens 20 beträgt.

2. Verzweigtes Polynukleotid nach Anspruch 1, dadurch **gekennzeichnet**, daß die erste einzelsträngige Polynukleotidsequenz von Interesse eine Analytnukleinsäure oder ein an eine Analytnukleinsäure gebundenes Polynukleotid ist und die zweite einzelsträngige Polynukleotidsequenz von Interesse ein markiertes Polynukleotid ist.

3. Verzweigtes Polynukleotid nach Anspruch 2, dadurch **gekennzeichnet**, daß es 15 bis 50 multifunktionelle Nukleotide umfaßt.

4. Verzweigtes Polynukleotid nach Anspruch 2, dadurch **gekennzeichnet**, daß das multifunktionelle Nukleotid die Formel 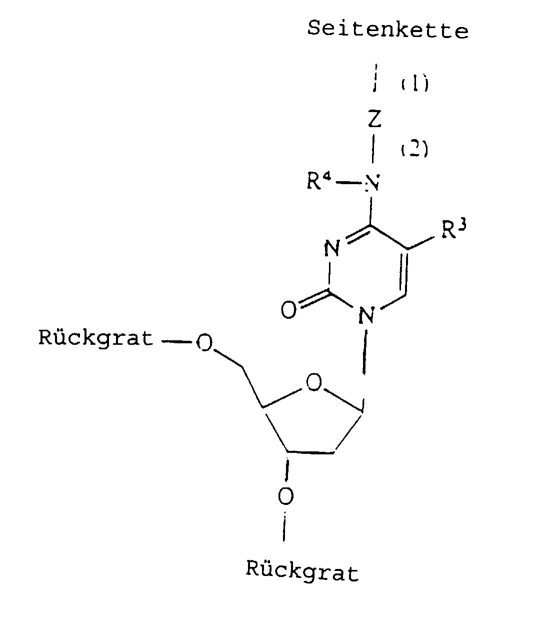 besitzt, in der R³ für Wasserstoff, Methyl, I, Br oder F steht, R⁴ für Wasserstoff oder Methyl steht, Z aus der Gruppe bestehend aus 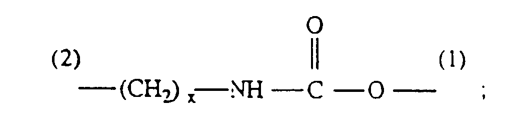 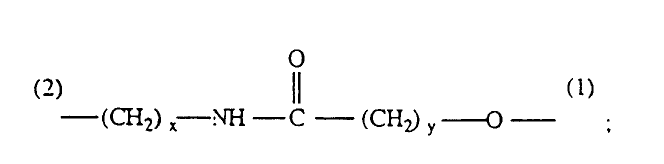 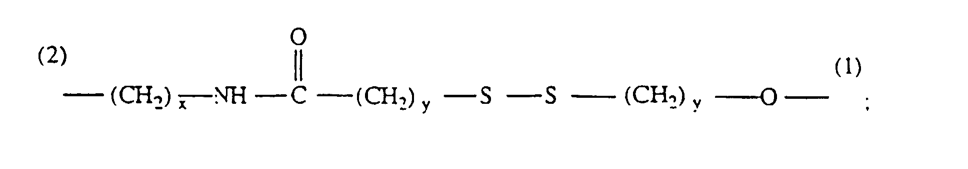 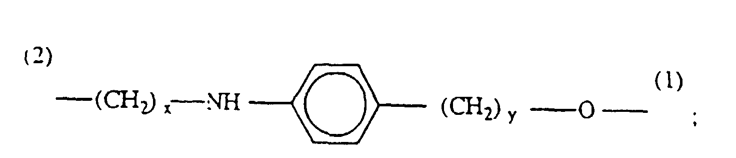
⁽²⁾―(CH₂―CH₂―O)ₓ―⁽¹⁾ ;
und
⁽²⁾―(CH₂)ₓ―O―⁽¹⁾,
worin x und y gleich oder unterschiedlich sein können und ganze Zahlen von 1 bis einschließlich 8 sind, ausgewählt ist.

5. Verzweigtes Polynukleotid nach Anspruch 2, dadurch **gekennzeichnet**, daß die ersten und zweiten einzelsträngigen Oligonukleotideinheiten jeweils 15 bis 50 Nukleotide lang sind.

6. Verzweigtes Polynukleotid nach Anspruch 2, dadurch **gekennzeichnet**, daß die Zahl der Wiederholungen in jeder Seitenkette 2 bis 10 beträgt.

7. Verzweigtes Polynukleotid nach Anspruch 1, dadurch **gekennzeichnet**, daß das verzweigte Polynukleotid die Formel 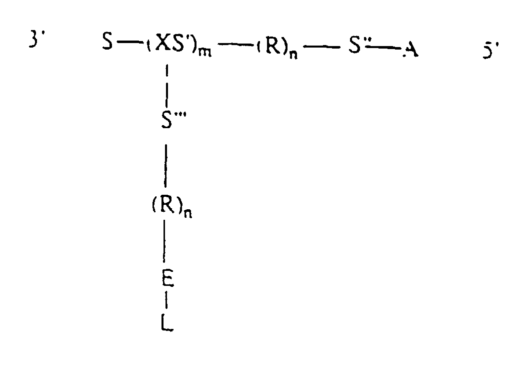 besitzt, in der S für ein erstes Spacersegment mit mindestens etwa 15 Nukleotiden steht, X für ein multifunktionelles Nukleotid, welches eine Verzweigungsstelle bereitstellt, steht, S' für ein Verzweigungsstellen-Spacer-segment mit 0 bis 15 Nukleotiden steht, m eine ganze Zahl gleich oder größer als 15 ist, R für ein spaltbares Linkermolekül steht, n 0 oder 1 ist, S" für ein zweites Spacersegment mit etwa 0 bis 10 Nukleotiden steht, A für ein Nukleotidsegment, welches an eine Analytnukleinsäure oder an eine Nukleinsäure, gebunden an einen Analyten, spezifisch hybridisieren kann, steht, S"' für ein drittes Spacersegment mit 0 bis 10 Nukleotiden steht, E für eine Oligonukleotidverlängerung von 5 bis 10 Nukleotiden steht und L für ein Segment steht, enthaltend 2 bis 10 Wiederholungen einer Nukleotidsequenz, welche spezifisch an eine markierte Oligonukleotidsonde hybridisieren kann.

8. Verzweigtes Polynukleotid nach Anspruch 7, dadurch **gekennzeichnet**, daß 5 eine Länge von 15 bis 50 Nukleotiden besitzt, X die Formel 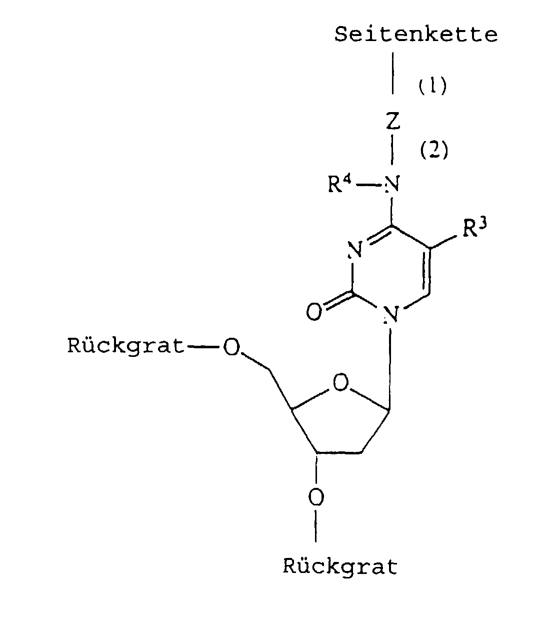 besitzt, in der R³ für Wasserstoff, Methyl, I, Br oder F steht, R⁴ für Wasserstoff oder Methyl steht, Z aus der Gruppe bestehend aus 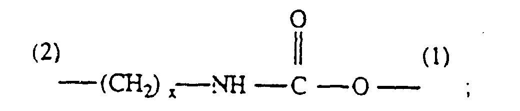 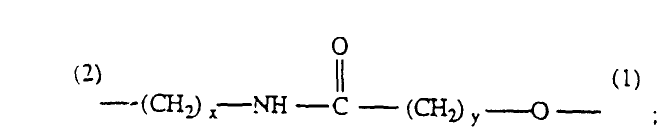 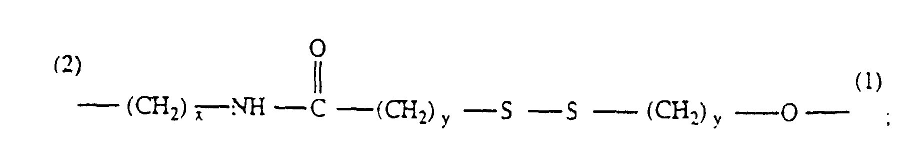 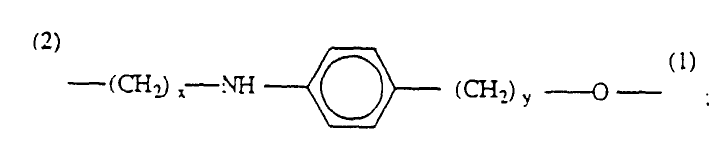
⁽²⁾―(CH₂―CH₂―O)ₓ―⁽¹⁾
und
⁽²⁾―(CH₂)ₓ―O―⁽¹⁾,
ausgewählt ist, worin x und y gleich oder unterschiedlich sein können und ganze Zahlen im Bereich von 1 bis einschließlich 8 ausgewählt sind, S" eine Länge von 5 bis 10 Nukleotiden besitzt und die Zahl der Wiederholungen in L 3 bis 6 beträgt.

9. Verzweigtes Polynukleotid nach Anspruch 8, dadurch **gekennzeichnet**, daß S für polyT steht.

10. Verzweigtes Polynukleotid nach Anspruch 8, dadurch **gekennzeichnet**, daß n 1 ist.

11. Verzweigtes Polynukleotid nach Anspruch 10, dadurch **gekennzeichnet**, daß das spaltbare Linkermolekül die Formel 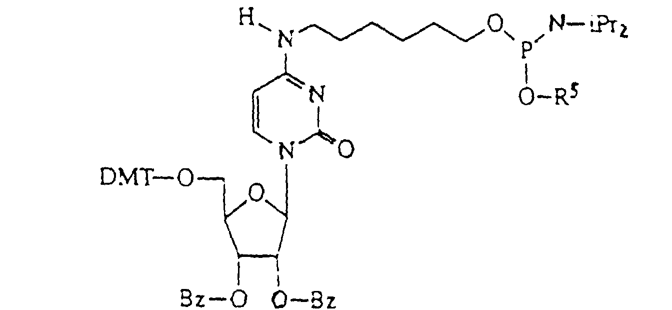 besitzt, in der DMT für Dimethoxytrityl steht, Bz für Benzoyl steht, R⁵ für Methyl oder β-Cyanoethyl steht und iPr für Isopropyl steht.

12. Verfahren zur Herstellung eines großen kammartig verzweigten Polynukleotids nach Anspruch 1, welches als ein Amplifikationsmultimeres bei einem Nukleinsäure-Hybridisierungsassay nützlich ist, umfassend:
(a) Synthetisieren eines einzelsträngigen Polynukleotidrückgrats, umfassend:
(i) mindestens 15 multifunktionelle Nukleotide, von denen jedes eine geschützte funktionelle Gruppe besitzt, welche als eine Stelle für eine Seitenketten-Nukleotidverlängerung dient, und
(ii) ein erstes Ligationsstellensegment;
(b) Entschützen der funktionellen Gruppen;
(c) Verlängern jeder der Stellen um mindestens etwa fünf Nukleotide, um zweite Ligationsstellensegmente bereitzustellen;
(d) Ligieren einer ersten einzelsträngigen Oligonukleotideinheit an die erste Ligationsstelle, wobei die erste einzelsträngige Oligonukleotideinheit eine erste einzelsträngige Nukleinsäuresequenz von Interesse binden kann, und
(e) Ligieren von zweiten einzelsträngigen Oligonukleotideinheiten an die zweiten Ligationsstellensegmente, wobei die zweiten einzelsträngigen Oligonukleotideinheiten Wiederholungen einer Sequenz umfassen, welche ein zweites einzelsträngiges Oligonukleotid von Interesse binden kann.

13. Verfahren nach Anspruch 12, dadurch **gekennzeichnet**, daß die Synthese und die Verlängerung mittels eines Festphasenprozesses ausgeführt werden, bei dem das 3'-Ende des Rückgrats an die feste Phase gebunden wird und das Rückgrat eine 3'-Leadersequenz von mindestens 15 Nukleotiden umfaßt.

14. Verfahren nach Anspruch 13, dadurch **gekennzeichnet**, daß die feste Phase Glas mit kontrollierten Poren von mindestens 2000 Å Porengröße ist.

15. Verfahren nach Anspruch 14, dadurch **gekennzeichnet**, daß die genannte Stelle zur Nukleotidverlängerung um 5 bis 10 Nukleotide verlängert wird.

16. Verfahren nach Anspruch 13, dadurch **gekennzeichnet**, daß das multifunktionelle Nukleotid die Formel 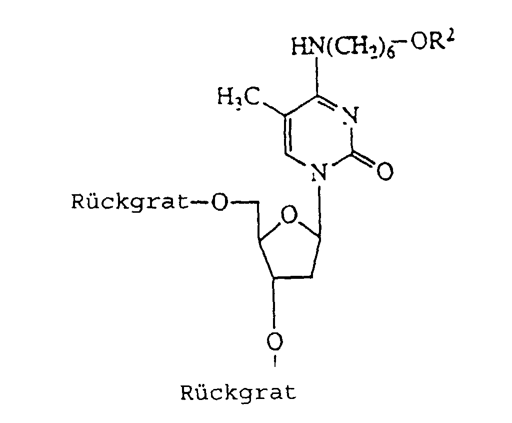 besitzt, in der R² für 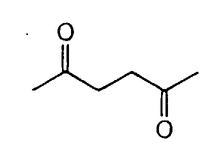 oder 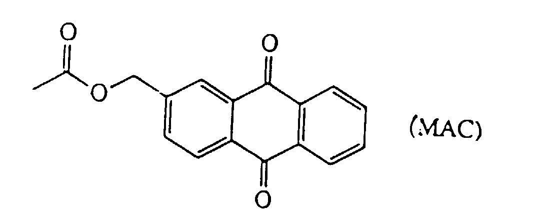 steht.

17. Verfahren zur Herstellung eines großen kammartig verzweigten Polynukleotids nach Anspruch 1, welches als ein Amplifikationsmultimeres bei einem Nukleinsäure-Hybridisierungsassay nützlich ist, umfassend:
(a) Synthetisieren eines einzelsträngigen Polynukleotid-Rückgrats, umfassend:
(i) mindestens 15 multifunktionelle Nukleotide, von denen jedes eine geschützte funktionelle Gruppe besitzt, welche als eine Stelle zur Seitenketten-Nukleotidverlängerung dient, und
(ii) eine erste einzelsträngige Oligonukleotideinheit, welche eine erste einzelsträngige Polynukleotidsequenz von Interesse spezifisch binden kann;
(b) Entschützen der funktionellen Gruppen;
(c) Verlängern jeder dieser Stellen um mindestens etwa 5 Nukleotide, um die Ligationsstellensegmente bereitzustellen, und
(d) Ligieren von zweiten einzelsträngigen Oligonukleotideinheiten an die Ligationsstellensegmente, wobei die zweiten einzelsträngigen Oligonukleotideinheiten Wiederholungen einer Sequenz umfassen, welche ein zweites einzelsträngiges Oligonukleotid von Interesse binden kann.

18. Verfahren nach Anspruch 17, dadurch **gekennzeichnet**, daß das multifunktionelle Nukleotid die Formel 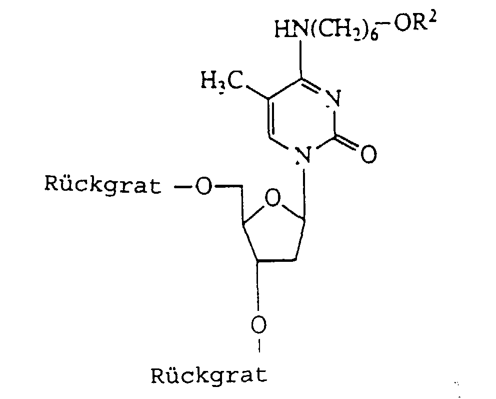 besitzt, in der R² für 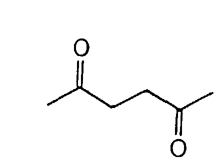 oder 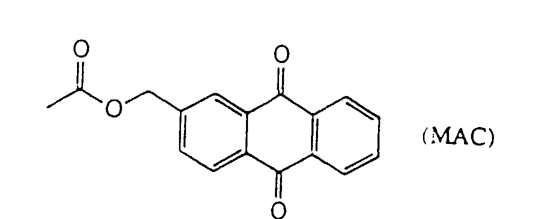 steht.

19. Verfahren nach Anspruch 18, dadurch **gekennzeichnet**, daß die Synthese und die Verlängerung mittels eines Festphasenprozesses ausgeführt werden, bei dem das 3'-Ende des Rückgrats an die feste Phase gebunden wird und das Rückgrat eine 3'-Spacersequenz von mindestens 15 Nukleotiden einschließt.

20. Verfahren nach Anspruch 18, dadurch **gekennzeichnet**, daß die feste Phase Glas mit kontrollierten Poren von mindestens 2000 Å Porengröße ist.

21. Verfahren nach Anspruch 18, dadurch **gekennzeichnet,** daß die Stelle zur Nukleotidverlängerung um 5 bis 10 Nukleotide verlängert wird.

22. Nukleinsäure-Hybridisierungsassay, bei dem eine Analytnukleinsäure an eine markierte Nukleinsäuresonde hybridisiert wird, und der dadurch gekennzeichnet ist, daß er das Hybridisieren des verzweigten Polynukleotids nach Anspruch 2 direkt oder indirekt an den Analyten über die erste einzelsträngige Oligonukleotideinheit und das Hybridisieren der markierten Nukleinsäuresonde an das verzweigte Polynukleotid über die zweite einzelsträngige Oligonukleotideinheit umfaßt.

23. Nukleinsäure-Hybridisierungsassay, bei dem eine Analytnukleinsäure an eine markierte Nukleinsäuresonde hybridisiert wird und der dadurch gekennzeichent ist, daß er das Hybridisieren des verzweigten Polynukleotids nach Anspruch 7 direkt oder indirekt an den Analyten über die erste einzelsträngige Oligonukleotideinheit und das Hybridisieren der markierten Nukleinsäuresonde an das verzweigte Polynukleotid über die zweite einzelsträngige Oligonukleotideinheit umfaßt.

24. Nukleinsäure-Hybridisierungsassay, bei dem:
(a) das verzweigte Polynukleotid nach Anspruch 2 über die erste Oligonukleotideinheit an eine einzelsträngige Analytnukleinsäure, gebunden an eine feste Phase oder an ein einzelsträngiges Oligonukleotid, gebunden an den Analyten, hybridisiert wird;
(b) nichtgebundenes verzweigtes Polynukleotid entfernt wird;
(c) einzelsträngiges markiertes Oligonukleotid an das verzweigte Polynukleotid über die zweiten Oligonukleotideinheiten hybridisiert wird;
(d) nichtgebundenes markiertes Oligonukleotid entfernt wird; und
(e) das Vorliegen der Markierung, gebunden an das verzweigte Polynukleotid, nachgewiesen wird.

25. Nukleinsäure-Hybridisierungsassay, bei dem:
(a) das verzweigte Polynukleotid nach Anspruch 7 über die erste Oligonukleotideinheit an einzelsträngige Analytnukleinsäure, gebunden an eine feste Phase, oder an ein einzelsträngiges Oligonukleotid, gebunden an den Analyten, hybridisiert wird;
(b) nichtgebundenes verzweigtes Polynukleotid entfernt wird;
(c) einzelsträngiges markiertes Oligonukleotid an das verzweigte Polynukleotid über die zweiten Oligonukleotideinheiten hybridisiert wird;
(d) nichtgebundenes markiertes Oligonukleotid entfernt wird; und
(e) das Vorliegen der Markierung, gebunden an das verzweigte Polynukleotid, nachgewiesen wird.

## Revendications

1. Un grand polynucléotide ramifié de type peigne comprenant :
(a) un squelette polynucléotidique ayant :
(i) au moins 15 nucléotides multifonctionnels, chacun de ceux-ci définissant un site de chaîne latérale et
(ii) une première unité oligonucléotidique simple brin qui est capable de se lier spécifiquement à une première séquence polynucléotidique simple brin d'intérêt ; et
(b) des chaînes latérales polynucléotidiques pendantes s'étendant à partir desdits nucléotides multifonctionnels, au moins 70 % de ceux-ci comprenant des répétitions d'une seconde unité oligonucléotidique simple brin qui est capable de se lier spécifiquement à une seconde séquence polynucléotidique simple brin d'intérêt, le nombre total de répétitions dans toutes les chaînes latérales étant d'au moins 20.

2. Le polynucléotide ramifié de la revendication 1 dans lequel la première séquence polynucléotidique simple brin d'intérêt est un acide nucléique constituant l'analyte ou un polynucléotide lié à l'acide nucléique constituant l'analyte et la seconde séquence polynucléotidique simple brin d'intérêt est un polynucléotide marqué.

3. Le polynucléotide ramifié de la revendication 2 dans lequel il y a 15 à 50 nucléotides multifonctionnels.

4. Le polynucléotide ramifié de la revendication 2 dans lequel le nucléotide multifonctionnel présente la formule 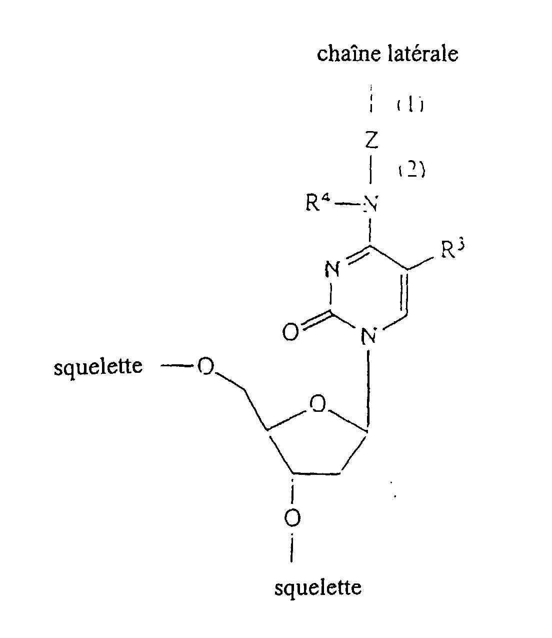 dans laquelle R³ est un hydrogène, un méthyle, I, Br ou F, R⁴ est un hydrogène ou un méthyle, Z est choisi dans le groupe constitué de 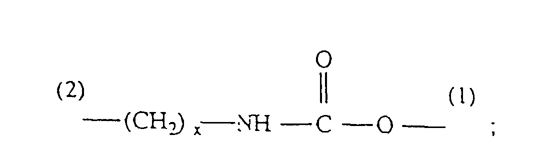 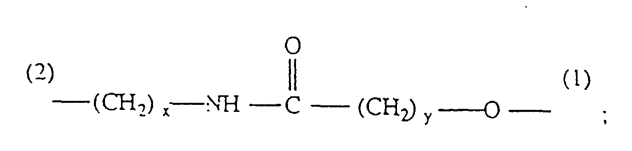 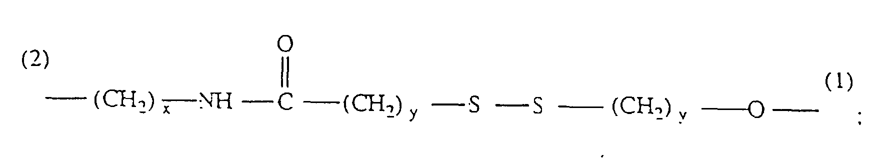 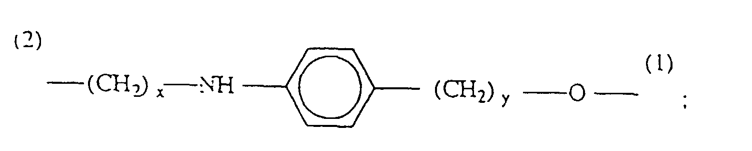
⁽²⁾―(CH₂―CH₂―O)ₓ―⁽¹⁾ ;
et
⁽²⁾―(CH₂)ₓ―O―⁽¹⁾,
dans lequel x et y peuvent être identiques ou différents et sont des nombres entiers dans la gamme de 1 à 8, inclusivement.

5. Le polynucléotide ramifié de la revendication 2 dans lequel les première et seconde unités oligonucléotidiques simple brin ont chacune une longueur de 15 à 50 nucléotides.

6. Le polynucléotide ramifié de la revendication 2 dans lequel le nombre de répétitions dans chaque chaîne latérale est de 2 à 10.

7. Le polynucléotide ramifié de la revendication 1 dans lequel le polynucléotide ramifié présente la formule 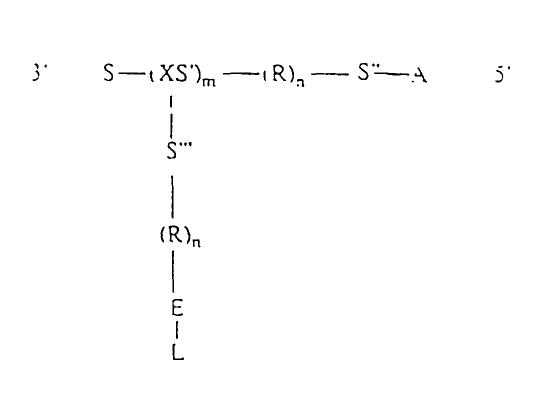 dans laquelle S est un premier segment espaceur d'au moins environ 15 nucléotides, X est un nucléotide multifonctionnel qui fournit un site de branchement, S' est un segment espaceur du site de branchement de O à 15 nucléotides, m est une nombre entier égal à 15 ou plus grand, R est une molécule linker qui peut être clivée, n est 0 ou 1, S" est un second segment espaceur d'environ 0 à 10 nucléotides, A est un segment nucléotidique qui est capable de s'hybrider spécifiquement avec l'acide nucléique constituant l'analyte ou l'acide nucléique lié à l'analyte, S"' est un troisième segment espaceur de 0 à 10 nucléotides, E est une extension oligonucléotidique de 5 à 10 nucléotides, et L est un segment contenant 2 à 10 répétitions d'une séquence nucléotidique qui est capable de s'hybrider spécifiquement avec une sonde oligonucléotidique marquée.

8. Le polynucléotide ramifié de la revendication 7 dans lequel S a une longueur de 15 à 50 nucléotides, X présente la formule 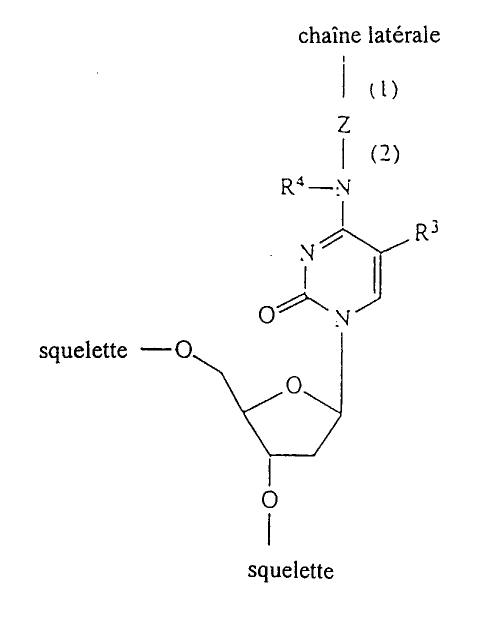 dans laquelle R³ est un hydrogène, un méthyle, I, Br ou F, R⁴ est un hydrogène ou un méthyle, Z est choisi dans le groupe constitué de 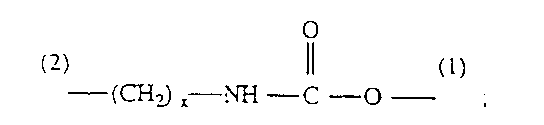 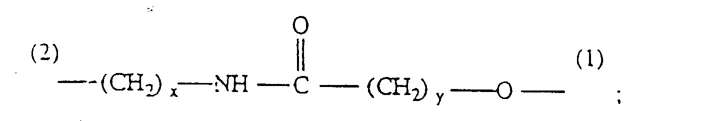 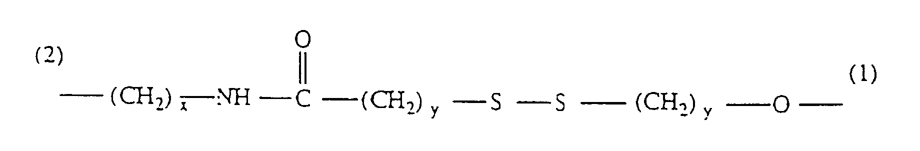 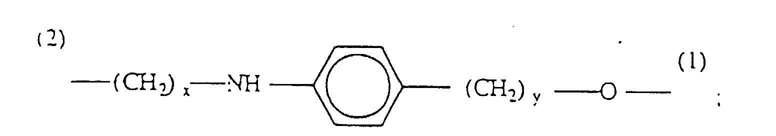
⁽²⁾―(CH₂―CH₂―O)ₓ―⁽¹⁾ ;
et
⁽²⁾―(CH₂)ₓ―O―⁽¹⁾,
Dans lequel x et y peuvent être identiques ou différents et sont des nombres entiers dans la gamme de 1 à 8 inclusivement S" a une longueur de 5 à 10 nucléotides, et le nombre de répétitions dans L est de 3 à 6.

9. Le polynucléotide ramifié de la revendication 8 dans lequel S est un polyT.

10. Le polynucléotide ramifié de la revendication 8 dans lequel n est 1.

11. Le polynucléotide ramifié de la revendication 10 dans lequel la molécule linker qui peut être clivée présente la formule 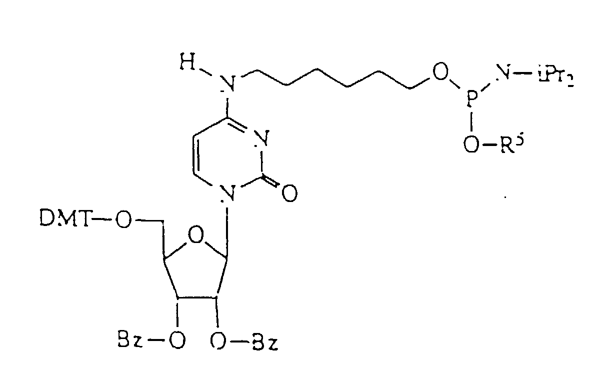 dans laquelle DMT représente un diméthoxytrityle, Bz représente un benzoyle, R⁵ représente un méthyle ou un β-cyanoéthyle, et iPr représente un isopropyle.

12. Un procédé destiné à la réalisation d'un grand polynucléotide ramifié de type peigne selon la revendication 1, utilisable comme multimère d'amplification dans un test d'hybridation d'acides nucléiques, comprenant :
(a) la synthèse d'un squelette polynucléotidique simple brin comprenant :
(i) au moins 15 nucléotides multifonctionnels, chacun de ceux-ci ayant un groupe fonctionnel protégé qui sert de site d'extension d'une chaîne latérale nucléotidique et
(ii) un premier segment constituant un site de ligature ;
(b) la déprotection desdits groupes fonctionnels ;
(c) l'extension de chacun desdits sites d'au moins environ cinq nucléotides pour fournir des segments constituant des seconds sites de ligature ;
(d) la ligature d'une première unité oligonucléotidique simple brin au premier site de ligature, ladite première unité oligonucléotidique simple brin étant capable de se lier à une première séquence d'acide nucléique simple brin d'intérêt ; et
(e) la ligature de secondes unités oligonucléotidiques simple brin aux segments constituant des seconds sites de ligature, lesdites secondes unités oligonucléotidiques simple brin comprenant des répétitions d'une séquence qui est capable de se lier à un second oligonucléotide simple brin d'intérêt.

13. Le procédé de la revendication 12 dans lequel ladite synthèse et ladite extension sont réalisées via un procédé en phase solide dans lequel l'extrémité 3' du squelette est fixée à la phase solide et le squelette comprend une séquence leader en 3' d'au moins 15 nucléotides.

14. Le procédé de la revendication 13 dans lequel la phase solide est un verre à porosité contrôlée avec des pores d'une taille d'au moins 2 000 Å.

15. Le procédé de la revendication 14 dans lequel ledit site d'extension nucléotidique est prolongé de 5 à 10 nucléotides.

16. Le procédé de la revendication 13 dans lequel le nucléotide multifonctionnel présente la formule 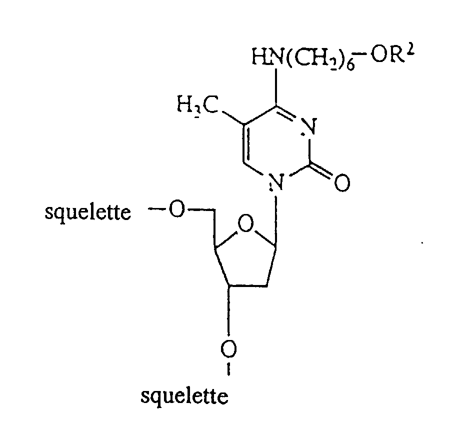 dans laquelle R² représente 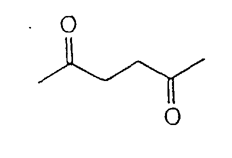 ou

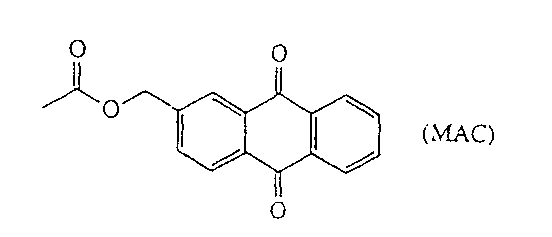

17. Un procédé destiné à la réalisation d'un grand polynucléotide ramifié de type peigne selon la revendication 1, utilisable comme multimère d'amplification dans un test d'hybridation d'acides nucléiques comprenant :
(a) la synthèse d'un squelette polynucléotidique simple brin comprenant :
(i) au moins 15 nucléotides multifonctionnels, chacun de ceux-ci ayant un groupe fonctionnel protégé qui sert de site d'extension d'une chaîne latérale nucléotidique et
(ii) une première unité oligonucléotidique simple brin qui est capable de se lier spécifiquement à une première séquence polynucléotidique simple brin d'intérêt ;
(b) la déprotection desdits groupes fonctionnels ;
(c) l'extension de chacun desdits sites d'au moins environ cinq nucléotides pour fournir des segments constituant des sites de ligature ; et
(d) la ligature de secondes unités oligonucléotidiques simple brin aux segments constituant des sites de ligature, lesdites secondes unités oligonucléotidiques simple brin comprenant des répétitions d'une séquence qui est capable de se lier à un second oligonucléotide simple brin d'intérêt.

18. Le procédé de la revendication 17 dans lequel le nucléotide multifonctionnel présente la formule 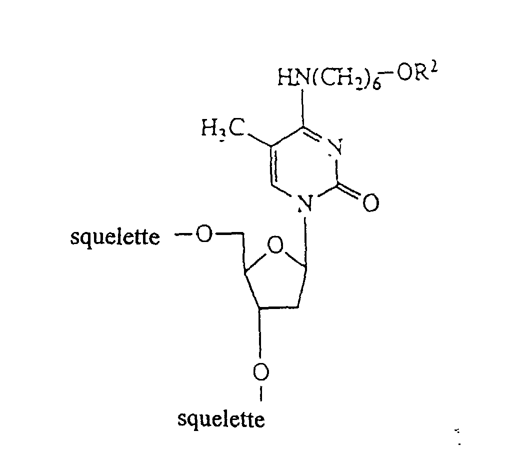 dans laquelle R² représente 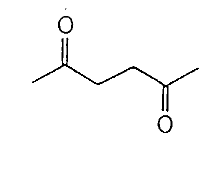 ou

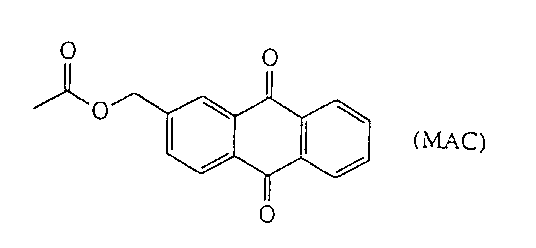

19. Le procédé de la revendication 18 dans lequel ladite synthèse et ladite extension sont réalisées via un procédé en phase solide dans lequel l'extrémité 3' du squelette est fixée à la phase solide et le squelette comprend une séquence espaceur en 3' d'au moins 15 nucléotides.

20. Le procédé de la revendication 18 dans lequel la phase solide est un verre à porosité contrôlée avec des pores d'une taille d'au moins 2 000 Å.

21. Le procédé de la revendication 18 dans lequel ledit site d'extension nucléotidique est prolongé de 5 à 10 nucléotides.

22. Un test d'hybridation d'acides nucléiques dans lequel l'acide nucléique constituant l'analyte est hybridé avec une sonde d'acide nucléique marquée, qui comprend l'hybridation du polynucléotide ramifié selon la revendication 2 directement ou indirectement avec l'analyte via la première unité oligonucléotidique simple brin et l'hybridation de la sonde d'acide nucléique marquée avec le polynucléotide ramifié via la seconde unité oligonucléotidique simple brin.

23. Un test d'hybridation d'acides nucléiques dans lequel l'acide nucléique constituant l'analyte est hybridé avec une sonde d'acide nucléique marquée, qui comprend l'hybridation du polynucléotide ramifié selon la revendication 7 directement ou indirectement avec l'analyte via la première unité oligonucléotidique simple brin et l'hybridation de la sonde d'acide nucléique marquée avec le polynucléotide ramifié via la seconde unité oligonucléotidique simple brin.

24. Un test d'hybridation d'acides nucléiques dans lequel :
(a) le polynucléotide ramifié selon la revendication 2 est hybridé via la première unité oligonucléotidique avec un acide nucléique simple brin constituant l'analyte lié à une phase solide ou avec un oligonucléotide simple brin lié à l'analyte ;
(b) le polynucléotide ramifié non lié est éliminé ;
(c) l'oligonucléotide marqué simple brin est hybridé avec le polynucléotide ramifié via les secondes unités oligonucléotidiques ;
(d) l'oligonucléotide marqué non lié est éliminé ; et
(e) la présence de marqueur lié au polynucléotide ramifié est détectée.

25. Un test d'hybridation d'acides nucléiques dans lequel :
(a) le polynucléotide ramifié selon la revendication 7 est hybridé via la première unité oligonucléotidique avec l'acide nucléique simple brin constituant l'analyte lié à une phase solide ou avec un oligonucléotide simple brin lié à l'analyte ;
(b) le polynucléotide ramifié non lié est éliminé ;
(c) l'oligonucléotide marqué simple brin est hybridé avec le polynucléotide ramifié via les secondes unités oligonucléotidiques ;
(d) l'oligonucléotide marqué non lié est éliminé ; et
(e) la présence de marqueur lié au polynucléotide ramifié est détectée.
